# EUROPEAN PATENT SPECIFICATION

(11) **EP 3 219 808 B1**
(45) Date of publication and mention of the grant of the patent: **01.01.2020**
(21) Application number: 15859983.7
(22) Date of filing: 13.11.2015
(51) Int. Cl.: C12Q 1/02, C07K 14/47, C12N 5/10, G01N 33/15, G01N 33/50, C12N 15/09

(54) **SCREENING METHOD USING INDUCED NEURONS**
SCREENING-VERFAHREN MIT INDUZIERTEN NEURONEN
PROCÉDÉ DE CRIBLAGE UTILISANT DES NEURONES INDUITS

(30) Priority: 13.11.2014 JP 2014230894
(43) Date of publication of application: 20.09.2017
(73) Proprietor: Kyoto University, Kyoto-shi, Kyoto 606-8501 (JP)
(72) Inventor: INOUE, Haruhisa, Kyoto-shi Kyoto 606-8501 (JP); IMAMURA, Keiko, Kyoto-shi Kyoto 606-8501 (JP)
(74) Representative: Hoffmann Eitle
(86) International application number: PCT/JP2015/082051
(87) International publication number: WO 2016/076435

(56) References cited:
- JP-A- 2010 538 611
- JP-A- 2013 503 201
- US-A1- 2008 138 811
- M. MELLONE ET AL: "Tau Pathology is Present In Vivo and Develops In Vitro in Sensory Neurons from Human P301S Tau Transgenic Mice: A System for Screening Drugs against Tauopathies", JOURNAL OF NEUROSCIENCE, vol. 33, no. 46, 13 November 2013 (2013-11-13), pages 18175-18189, XP55164189, ISSN: 0270-6474, DOI: 10.1523/JNEUROSCI.4933-12.2013
- HELEN FONG ET AL: "Genetic Correction of Tauopathy Phenotypes in Neurons Derived from Human Induced Pluripotent Stem Cells", STEM CELL REPORTS, vol. 1, no. 3, 10 September 2013 (2013-09-10), pages 226-234, XP55441675, United States ISSN: 2213-6711, DOI: 10.1016/j.stemcr.2013.08.001
- LI-WEN KO ET AL: "Cellular Models for Tau Filament Assembly", MOLECULAR AND CHEMICAL NEUROPATHOLOGY., vol. 19, no. 3, 1 December 2002 (2002-12-01), pages 309-316, XP55467475, US ISSN: 1044-7393, DOI: 10.1385/JMN:19:3:309
- MARC EHRLICH ET AL: "Distinct Neurodegenerative Changes in an Induced Pluripotent Stem Cell Model of Frontotemporal Dementia Linked to Mutant TAU Protein", STEM CELL REPORTS, vol. 5, no. 1, 1 July 2015 (2015-07-01), pages 83-96, XP55467602, United States ISSN: 2213-6711, DOI: 10.1016/j.stemcr.2015.06.001
- SULLIVAN SARAH E ET AL: "Induced pluripotent stem cells as a discovery tool for Alzheimer?s disease", BRAIN RESEARCH, ELSEVIER, AMSTERDAM, NL, vol. 1656, 13 October 2015 (2015-10-13), pages 98-106, XP029896325, ISSN: 0006-8993, DOI: 10.1016/J.BRAINRES.2015.10.005
- OBERSTEIN TIMO JAN ET AL: "Astrocytes and microglia but not neurons preferentially generate N-terminally truncated A[beta] pept", NEUROBIOLOGY OF DISEASE, ELSEVIER, AMSTERDAM, NL, vol. 73, 7 September 2014 (2014-09-07), pages 24-35, XP029097216, ISSN: 0969-9961, DOI: 10.1016/J.NBD.2014.08.031
- SE HOON CHOI ET AL: "A three-dimensional human neural cell culture model of Alzheimer's disease", NATURE, vol. 515, no. 7526, 12 October 2014 (2014-10-12), pages 274-278, XP55394768, GB ISSN: 0028-0836, DOI: 10.1038/nature13800
- FONG H ET AL.: 'Genetic Correction of Tauopathy Phenotypes in Neurons Derived from Human Induced Pluripotent Stem Cells' STEM CELL REPORTS vol. 1, no. 3, 10 September 2013, pages 226 - 234, XP055441675 DOI: 10.1016/J.STEMCR.2013.08.001
- HARGUS G ET AL.: 'Using inducedpluripotent stem cells to model frontotemporal dementia' CLIN. NEUROPATHOL vol. 32, no. 5, 2013, page 422, XP009502912
- MELLONE M ET AL.: 'Tau pathology is present in vivo and develops in vitro in sensory neurons from human P301S tau transgenic mice: a system for screening drugs against tauopathies' J. NEUROSCI. vol. 33, no. 46, 13 November 2013, pages 18175 - 18189, XP055164189 DOI: 10.1523/JNEUROSCI.4933-12.2013
- BERGER Z ET AL.: 'Accumulation of pathological tau species and memory loss in a conditional model of tauopathy' J. NEUROSCI. vol. 27, no. 14, 04 April 2007, pages 3650 - 3662, XP055441682 DOI: 10.1523/JNEUROSCI.0587-07.2007
- GÓMEZ-RAMOS A ET AL.: 'Extracellular tau promotes intracellular calcium increase through M1 and M3 muscarinic receptors in neuronal cells' MOL. CELL.NEUROSCI vol. 37, no. 4, 15 December 2007, pages 673 - 681, XP022576897
- GÓMEZ-RAMOS A ET AL.: 'Extracellular tau is toxic to neuronal cells' FEBS LETT vol. 580, no. 20, 04 September 2006, pages 4842 - 4850, XP005623773 DOI: 10.1016/J.FEBSLET.2006.07.078
- ROCHER AB ET AL.: 'Structural and functional changes in tau mutant mice neurons are not linked to the presence of NFTs' EXP. NEUROL. vol. 223, no. 2, 01 June 2010, pages 385 - 393, XP027038380
- ZHANG Y ET AL.: 'Rapid single-step induction of functional neurons from human pluripotent stem cells' NEURON vol. 78, no. 5, 05 June 2013, pages 785 - 798, XP028562742 DOI: 10.1016/J.NEURON.2013.05.029

### SEQUENCE LISTING

<110> Kyoto University
<120> Method of Screening Using Induced Neurons
<130> CR0011
<160> 4
<170> PatentIn version 3.5
<210> 1
   <211> 441
   <212> PRT
   <213> Homo sapiens
<220>
   <223> Human Tau isoform 2 (2N4R)
<400> 1
<210> 2
   <211> 5811
   <212> DNA
   <213> Homo sapiens
<220>
   <221> mRNA
<220>
   <221> CDS
   <222> 323..1648
   <223> /gene="MAPT"
<220>
   <221> exon
   <222> 1145..1237
   <223> /number=10 /gene="MAPT"
<220>
   <221> exon
   <222> 1238..1319
   <223> /number=11 /gene="MAPT"
<220>
   <223> Human MAPT transcript variant 2 (Tau 2N4R)
<400> 2
<210> 3
   <211> 3780
   <212> DNA
   <213> Homo sapiens
<220>
   <221> intron
   <222> 1..3780
   <223> /number=10 /gene="MAPT"
<220>
   <223> Human MAPT gene intron 10
<400> 3
   gtgagtacct tcacacgtcc catgcgccgt gctgtggctt gaattattag gaagtggtgt 60
   gagtgcgtac acttgcgaga cactgcatag aataaatcct tcttgggctc tcaggatctg 120
   gctgcgacct ctgggtgaat gtagcccggc tccccacatt cccccacacg gtccactgtt     180
   cccagaagcc ccttcctcat attctaggag ggggtgtccc agcatttctg ggtcccccag     240
   cctgcgcagg ctgtgtggac agaatagggc agatgacgga ccctctctcc ggaccctgcc     300
   tgggaagctg agaataccca tcaaagtctc cttccactca tgcccagccc tgtccccagg     360
   agccccatag cccattggaa gttgggctga aggtggtggc acctgagact gggctgccgc     420
   ctcctccccc gacacctggg caggttgacg ttgagtggct ccactgtgga caggtgaccc     480
   gtttgttctg atgagcggac accaaggtct tactgtcctg ctcagctgct gctcctacac     540
   gttcaaggca ggagccgatt cctaagcctc cagcttatgc ttagcctgcg ccaccctctg     600
   gcagagactc cagatgcaaa gagccaaacc aaagtgcgac aggtccctct gcccagcgtt     660
   gaggtgtggc agagaaatgc tgcttttggc ccttttagat ttggctgcct cttgccagga     720
   gtggtggctc gtgcctgtaa ttccagcact ttgggagact aaggcgggag gttcgcttga     780
   gcccaggagt tcaagaccag cctgggcaac aatgagaccc ctgtgtctac aaaaagaatt     840
   aaaattagcc aggtgtggtg gcacgcacct gtagtcccag ctacttggga ggctgaggtg     900
   ggaggattgc ctgagtccgg gaggcggaag ttgcaaggag ccatgatcgc gccactgcac     960
   ttcaacctag gcaacagagt gagactttgt ctcaaaaaac aatcatataa taattttaaa    1020
   ataaatagat ttggcttcct ctaaatgtcc ccggggactc cgtgcatctt ctgtggagtg    1080
   tctccgtgag attcgggact cagatcctca agtgcaactg acccacccga taagctgagg    1140
   cttcatcatc ccctggccgg tctatgtcga ctgggcaccc gaggctcctc tcccaccagc    1200
   tctcttggtc agctgaaagc aaactgttaa caccctgggg agctggacgt atgagaccct    1260
   tggggtggga ggcgttgatt tttgagagca atcacctggc cctggctggc agtaccggga    1320
   cactgctgtg gctccggggt gggctgtctc cagaaaatgc ctggcctgag gcagccaccc    1380
   gcatccagcc cagagggttt attcttgcaa tgtgctgctg cttcctgccc tgagcacctg    1440
   gatcccggct tctgccctga ggccccttga gtcccacagg tagcaagcgc ttgccctgcg    1500
   gctgctgcat ggggctaact aacgcttcct caccagtgtc tgctaagtgt ctcctctgtc    1560
   tcccacgccc tgctctcctg tccccccagt ttgtctgctg tgaggggaca gaagaggtgt    1620
   gtgccgcccc cacccctgcc cgggcccttg ttcctgggat tgctgttttc agctgtttga    1680
   gctttgatcc tggttctctg gcttcctcaa agtgagctcg gccagaggag gaaggccatg    1740
   tgctttctgg ttgaagtcaa gtctggtgcc ctggtggagg ctgtgctgct gaggcggagc    1800
   tggggagaga gtgcacacgg gctgcgtggc caacccctct gggtagctga tgcccaaaga    1860
   cgctgcagtg cccaggacat ctgggacctc cctggggccc gcccgtgtgt cccgcgctgt    1920
   gttcatctgc gggctagcct gtgacccgcg ctgtgctcgt ctgcgggcta gcctgtgtcc    1980
   cgcgctctgc ttgtctgcgg tctagcctgt gacctggcag agagccacca gatgtcccgg    2040
   gctgagcact gccctctgag caccttcaca ggaagccctt ctcctggtga gaagagatgc 2100
   cagcccctgg catctggggg cactggatcc ctggcctgag ccctagcctc tccccagcct 2160
   gggggcccct tcccagcagg ctggccctgc tccttctcta cctgggaccc ttctgcctcc 2220
   tggctggacc ctggaagctc tgcagggcct gctgtccccc tccctgccct ccaggtatcc 2280
   tgaccaccgg ccctggctcc cactgccatc cactcctctc ctttctggcc gttccctggt 2340
   ccctgtccca gcccccctcc ccctctcacg agttacctca cccaggccag agggaagagg 2400
   gaaggaggcc ctggtcatac cagcacgtcc tcccacctcc ctcggccctg gtccaccccc 2460
   tcagtgctgg cctcagagca cagctctctc caagccaggc cgcgcgccat ccatcctccc 2520
   tgtcccccaa cgtccttgcc acagatcatg tccgccctga cacacatggg tctcagccat 2580
   ctctgcccca gttaactccc catccataaa gagcacatgc cagccgacac caaaataatt 2640
   cgggatggtt ccagtttaga cctaagtgga aggagaaacc accacctgcc ctgcaccttg 2700
   ttttttggtg accttgataa accatcttca gccatgaagc cagctgtctc ccaggaagct 2760
   ccagggcggt gcttcctcgg gagctgactg ataggtggga ggtggctgcc cccttgcacc 2820
   ctcaggtgac cccacacaag gccactgctg gaggccctgg ggactccagg aatgtcaatc 2880
   agtgacctgc cccccaggcc ccacacagcc atggctgcat agaggcctgc ctccaaggga 2940
   cctgtctgtc tgccactgtg gagtccctac agcgtgcccc ccacagggga gctggttctt 3000
   tgactgagat cagctggcag ctcagggtca tcattcccag agggagcggt gccctggagg 3060
   ccacaggcct cctcatgtgt gtctgcgtcc gctcgagctt actgagacac taaatctgtt 3120
   ggtttctgct gtgccaccta cccaccctgt tggtgttgct ttgttcctat tgctaaagac 3180
   aggaatgtcc aggacactga gtgtgcaggt gcctgctggt tctcacgtcc gagctgctga 3240
   actccgctgg gtcctgctta ctgatggtct ttgctctagt gctttccagg gtccgtggaa 3300
   gcttttcctg gaataaagcc cacgcatcga ccctcacagc gcctcccctc tttgaggccc 3360
   agcagatacc ccactcctgc ctttccagca agatttttca gatgctgtgc atactcatca 3420
   tattgatcac ttttttcttc atgcctgatt gtgatctgtc aatttcatgt caggaaaggg 3480
   agtgacattt ttacacttaa gcgtttgctg agcaaatggg gattgaagac actccagtcc 3540
   cacagtcccc agctcccctg gggcagggtt ggcagaattt cgacaacaca tttttccacc 3600
   ctgactagga tgtgctcctc atggcagctg ggaaccactg tccaataagg gcctgggctt 3660
   acacagctgc ttctcattga gttacaccct taataaaata atcccatttt atcctttttg 3720
   tctctctgtc ttcctctctc tctgcctttc ctcttctctc tcctcctctc tcatctccag 3780
<210> 4
   <211> 272
   <212> PRT
   <213> Homo sapiens
<220>
   <223> Human Neurogenin2
<400> 4
   Met Phe Val Lys Ser Glu Thr Leu Glu Leu Lys Glu Glu Glu Asp Val
   1               5                   10                  15
   Leu Val Leu Leu Gly Ser Ala Ser Pro Ala Leu Ala Ala Leu Thr Pro
               20                  25                  30
   Leu Ser Ser Ser Ala Asp Glu Glu Glu Glu Glu Glu Pro Gly Ala Ser
           35                  40                  45
   Gly Gly Ala Arg Arg Gln Arg Gly Ala Glu Ala Gly Gln Gly Ala Arg
       50                  55                  60
   Gly Gly Val Ala Ala Gly Ala Glu Gly Cys Arg Pro Ala Arg Leu Leu
   65                  70                  75                  80
   Gly Leu Val His Asp Cys Lys Arg Arg Pro Ser Arg Ala Arg Ala Val
                   85                  90                  95
   Ser Arg Gly Ala Lys Thr Ala Glu Thr Val Gln Arg Ile Lys Lys Thr
               100                 105                 110
   Arg Arg Leu Lys Ala Asn Asn Arg Glu Arg Asn Arg Met His Asn Leu
           115                 120                 125
   Asn Ala Ala Leu Asp Ala Leu Arg Glu Val Leu Pro Thr Phe Pro Glu
       130                 135                 140
   Asp Ala Lys Leu Thr Lys Ile Glu Thr Leu Arg Phe Ala His Asn Tyr
   145                 150                 155                 160
   Ile Trp Ala Leu Thr Glu Thr Leu Arg Leu Ala Asp His Cys Gly Gly
                   165                 170                 175
   Gly Gly Gly Gly Leu Pro Gly Ala Leu Phe Ser Glu Ala Val Leu Leu
               180                 185                 190
   Ser Pro Gly Gly Ala Ser Ala Ala Leu Ser Ser Ser Gly Asp Ser Pro
           195                 200                 205
   Ser Pro Ala Ser Thr Trp Ser Cys Thr Asn Ser Pro Ala Pro Ser Ser
       210                 215                 220
   Ser Val Ser Ser Asn Ser Thr Ser Pro Tyr Ser Cys Thr Leu Ser Pro
   225                 230                 235                 240
   Ala Ser Pro Ala Gly Ser Asp Met Asp Tyr Trp Gln Pro Pro Pro Pro
                   245                 250                 255
   Asp Lys His Arg Tyr Ala Pro His Leu Pro Ile Ala Arg Asp Cys Ile
               260                 265                 270
<210> 5
   <211> 22
   <212> RNA
   <213> Artificial Sequence
<220>
   <223> gRNA
<400> 5
   gcaacgucca guccaagugu gg 22

## Description

### TECHNICAL FIELD

The present invention relates to a method for screening a prophylactic or therapeutic agent for tauopathy. More specifically, the present invention relates to the said agent using neurons generated from pluripotent stem cells.

### BACKGROUND OF THE INVENTION

Microtubule-associated protein tau (MAPT) is a microtubule-associated protein expressed mainly in the nervous system. This protein performs the promotion of tubulin polymerization and the stabilization of microtubules and contributes to the construction and maintenance of nerve axons. The tau protein is encoded by MAPT gene and expressed as 6 types of isoforms by alternative splicing in the human brain. Particularly, the alternative splicing of exon 10 is important. When the exon is spliced, 3R tau (3-repeat tau) appears which has 3 repeat sequences involved in microtubule binding. When the exon is not spliced, 4R tau (4-repeat tau) appears which has 4 such sequences. All of the isoforms are known to lose the ability to bind to microtubules and be self-aggregated through hyperphosphorylation (Non-Patent Documents 1 and 2).

In Alzheimer's disease (hereinafter, referred to as AD) or frontotemporal dementia with parkinsonism linked to chromosome 17 (hereinafter, referred to as FTDP-17), aggregates (neurofibrillary tangles; hereinafter, referred to as NFT) composed mainly of paired helical filaments (hereinafter, referred to as PHF) consisting of phosphorylated tau are formed in brain neurons, and it is considered that the NFT and the process of formation thereof are deeply involved in the development of these diseases (Non-Patent Documents 1 and 2). It is further known that the distribution of neurons having the NFT is diffused in the brain as the diseases progress. For example, in earliest stage AD patients, the NTF-containing neurons are detected in the entorhinal area. In middle stage AD patients, the NTF-containing neurons are also detected in the temporal lobe. In late stage AD patients with advanced dementia, the NTF-containing neurons are detected in the entire association area of the cerebral cortex. This phenomenon is called "propagation of tau toxicity" or "propagation of tau-mediated neurodegeneration" and is considered to occur because tau toxicity is propagated among neurons through an unknown vehicle (Non-Patent Document 3).

As mentioned above, neurodegenerative disease that involves the aggregation and intracellular accumulation of tau and is developed due to the process of tau aggregation is called tauopathy, and in japan alone, 2,000,000 or more patients suffer from this disease.

FTDP-17 is a neurodegenerative disease linked to MAPT gene loci. A large number of MAPT gene mutations have been identified from FTDP-17 families. These mutations can be divided into mutations to form mutant tau proteins and mutations to elevate a 4-repeat tau/3-repeat tau expression ratio (Non-Patent Document 1). Many transgenic mice that manifest pathological changes similar to those of tauopathy patients, such as hyperphosphorylation, oligomerization, or aggregation of mutant tau, neurological dysfunction (decrease in synaptic density, reduction in nerve excitability, memory disorder, etc.), and neurodegeneration, have been obtained so far by the transfection of mice with mutant MAPT genes having the former mutations (Non-Patent Documents 1, 4, and 5). Furthermore, results of analysis using these tauopathy mouse models support the hypothesis that intracellular toxicity exerted by tau aggregates having a lower molecular weight (i.e., tau oligomers) than that of PHF causes neurological dysfunction and neurodegeneration (Non-Patent Documents 1, 2, 4, and 5).

On the other hand, the latter mutations are mutations to inhibit the splicing of exon 10, and the great majority of the mutations occur within intron 10. In the human adult brain, the expression level of 4-repeat tau and the expression level of 3-repeat tau are usually almost the same. In the brains of FTDP-17 patients having the latter mutations, the 4-repeat tau/3-repeat tau expression ratio is elevated, and hyperphosphorylated 4-repeat tau accumulates as a main component of NFT according to the reports (Non-Patent Documents 6 to 8). Nonetheless, neither NFT formation nor neurodegeneration basically occurs even if 4-repeat tau is overexpressed by the transfection of mice with its cDNA. Therefore, the mechanism under which the latter MAPT gene mutations cause tauopathy has been almost unknown. In response to this problem, transgenic mice harboring tau cDNA having intron 9 and mutant intron 10 have been prepared recently. It has been reported that in these mice, tau hyperphosphorylation, neurological dysfunction, NFT formation, and neuronal death occur as the 4-repeat tau/3-repeat tau expression ratio is elevated with aging (Non-Patent Document 9). Accordingly, as for these MAPT gene mutations, although the mechanism under which the elevation of the 4-repeat tau/3-repeat tau expression ratio causes tau hyperphosphorylation or aggregation is unknown, the subsequent process seems to cause neurodegeneration under the same mechanism as in the former MAPT gene mutations.

On the basis of these findings, compounds capable of inhibiting tau phosphorylation or aggregation, or cytotoxicity mediated by aggregated tau have been energetically searched for, in the field of drug discovery related to tauopathy, using various *in vitro* and/or cultured cell-based screening systems.

Among them, for example, a method which involves contacting tau or a tau fragment (core fragment containing a site involved in aggregation) bound with a solid phase with test substances and screening for a candidate compound with tau-tau association inhibition as an index (Patent Document 1), a method which involves contacting oligomerized tau with test substances in the presence of heparin *in vitro* and screening for a candidate by using the ability to bind to the tau oligomer and inhibition of aggregation of the oligomers as indexes (Patent Document 2), and a method using phosphorylation inhibitory activity against a particular tau-phosphorylating enzyme as an index (e.g., Patent Document 3) are known as the *in vitro* screening systems.

However, compounds obtained by such *in vitro* screening systems are accompanied by the possibility (risk) that the compounds cannot overcome problems such as cytotoxicity or cell membrane permeability. Therefore, the cultured cell-based screening systems, which can also evaluate these problems, tend to be preferred.

Methods which involve adding test substances to a medium of cultured cells caused to overexpress tau or the core fragment, and screening for a candidate compound by using the ability to bind to the core fragment (Patent Document 4), tau aggregation inhibition or inhibition of cell degeneration or cell death (Patent Document 1), or inhibition of reduction in proteasome function (Patent Document 5) as an index have been developed as the screening systems using cultured cells. Also, there is a report on a method which involves administering test substances to cells caused to overexpress tau and a tau-phosphorylating enzyme (protein kinase X linked), and screening for a candidate by using inhibitory effects on tau phosphorylation, tau aggregation, cell degeneration, or cell death as an index (e.g., Patent Document 6). These screening methods generally employ a cell line or neurons differentiated by induction from the cell line. By contrast, a method using a rat brain-derived primary neuron culture system is also known (Patent Document 6).

However, all of these methods are intended to screen for compounds capable of alleviating toxicity produced in the inside of cells (i.e., intracellular toxicity of tau) forced to overexpress phosphorylated tau or aggregated tau.

As mentioned above, the degree of progression of dementia in AD patients correlates with the level of propagation of tau toxicity. In general, dementia becomes severe after neurodegeneration arrives at the association area of the cerebral cortex (Non-Patent Document 2). Accordingly, there is the possibility that even dementia that has already manifest itself in a patient does not become severe if the subsequent propagation of neurodegeneration can be sufficiently blocked. Thus, the blocking or delay of propagation of neurodegeneration has been recognized as being of very great clinical importance. However, any method for screening for a agent that exerts such effects has not been reported. This is because an actual substance responsible for the propagation is unknown, and furthermore, a cell culture system that reproduces the propagation of tau-mediated neurodegeneration is absent.

Hence, a method for screening for a compound that can not only block the intracellular toxicity of tau but also block the intercellular propagation of neurodegeneration has been desired in the field of tauopathy drug discovery.

Mellone et al. (The journal of Neuroscience, 2013; 33(46):18175-18189) describe that dorsal root ganglion (DRG) neurons in transgenic mice expressing human P301S tau (P301S-htau) develop tau pathology similar to that found in brain and spinal cord and that a significant reduction in mechanosensation occurs before detectable fibrillar tau formation. DRG neuronal cultures established from adult P301S-htau mice at different ages retained the pattern of aberrant tau found in vivo.

US 2008/138811 relates to modified brain slice cultures and assay systems based thereon for screening agents capable of modulating etiopathology of neuronal cells, in particular in the context of Alzheimer's disease related brain lesions.

Fong et al. (Stem Cell Reports, 2013; 1(3):226-234) report a human neuronal model of tauopathy derived from induced pluripotent stem cells (iPSCs) carrying a TAU-A152T mutation. The A152T mutation increased TAU fragmentation and phosphorylation, leading to neurodegeneration and especially axonal degeneration.

Ko et al. (Journal of Molecular Neuroscience, 2002; 19(3):311-316) describe conditional transfectants from human neuroglioma [H4] and neuronal [BE(2)-M17D] cells whose transgenic expression of wild-type or mutant tau via inducible expression mechanisms leads to tau aggregation and filament assembly.

Oberstein et al. (Neurobiology of Disease, 2015; 73:24-35) describe that Aβ peptides released from astrocytes and microglia include high proportions of N-terminally modified Aβ peptides. The inhibition of BACE1 reduced the amount of Aβ 1-x in cell culture supernatants but not the amount of Aβ 2-x. Immunostaining with antibodies against tau is described.

Choi et al. (Nature, 2014; 515(7526):274-278) report that FAD mutations in β-amyloid precursor protein and presenilin 1 are able to induce robust extracellular deposition of amyloid-B in a human neural stem-cell-derived 3D culture system. The 3D-differentiated neuronal cells expressing FAD mutations exhibited high levels of detergent-resistant, silver-positive aggregates of phosphorylated tau in the soma and neurites, as well as filamentous tau, as detected by immunoelectron microscopy. Inhibition of amyloid-B generation with β- or y-secretase inhibitors not only decreased amyloid-B pathology, but also attenuated tauopathy.

### PRIOR ART DOCUMENTS

### PATENT DOCUMENTS

[Patent Document 1] WO1996/030766
[Patent Document 2] WO2013/051266
[Patent Document 3] WO2007/088400
[Patent Document 4] WO2002/059150
[Patent Document 5] Japanese Unexamined Patent Application Publication No. 2007-209227
[Patent Document 6] WO2009/101942
[Patent Document 7] WO2014/148646

### NON-PATENT DOCUMENTS

[Non-Patent Document 1] Lee V.M., et al, Annu. Rev. Neurosci., 24:1121-159, 2001
[Non-Patent Document 2] Akihiko Takashima, Human Science, 24:22-25, 2013
[Non-Patent Document 3] Clavaguera F., et al, Nat. Cell Biol., 11:909-13, 2009
[Non-Patent Document 4] Denk F., et al, Neurobiol. Aging, 30:1-13, 2009
[Non-Patent Document 5] Berger Z., et al, J. Neurosci., 27:3650-62, 2007
[Non-Patent Document 6] Zhou J., et al, BMC Neurosci., 9(Suppl.2):S10, 2008
[Non-Patent Document 7] Grover A., et al, J. Biol. Chem., 274:15134-43, 1999
[Non-Patent Document 8] Hutton M., et al, Nature, 393:702-5, 1998
[Non-Patent Document 9] Umeda T., et al, The American Journal of Pathology, 183:210-25, 2013
[Non-Patent Document 10] Zhang Y., et al, Neuron, 78:785-98, 2013
[Non-Patent Document 11] Tanaka A., et al, PLoS One, 8:e61540, 2013
[Non-Patent Document 12] Patterson K., et al, J. Biol. Chem., 286:23063-76, 2011
[Non-Patent Document 13] Ward S.M., et al, J. Alzheimers Dis., 37:593-602, 2014

### DISCLOSURE OF THE INVENTION

### PROBLEM TO BE SOLVED BY THE INVENTION

An object of the present invention is to provide a method for screening for a prophylactic or therapeutic agent for tauopathy capable of blocking the propagation of tau-mediated neurodegeneration.

### MEANS TO SOLVE THE PROBLEM

In order to solve the problems of the conventional techniques, the present inventors have prepared induced pluripotent stem cells from mouse and human cells having a mutant MAPT gene and allowed the induced pluripotent stem cells to differentiate into neurons by the expression of a foreign neurogenin 2 gene. These neurons have been found to be excellent tauopathy cell models that spontaneously exhibit typical pathological conditions of tauopathy, such as tau hyperphosphorylation and oligomerization, and decrease in synaptic density, and cause cell death.

The present inventors have further found that in the tauopathy model cells, both of a spontaneous firing rate and a firing rate are increased, and these cells secrete a tau oligomer into a medium in a manner dependent on neural activity. Furthermore, the secreted tau oligomer has been found to specifically bind to lipid raft on the cell membranes of normal neurons and increase the excitability of the normal neurons to induce tauopathy-like cell death.

Specifically, the present inventors have revealed that the tau oligomer secreted by the tauopathy model cells is principally responsible for the propagation of tau-mediated neurodegeneration, leading to the completion of the present invention.

The present invention encompasses the following:
[1] A method for screening for a prophylactic or therapeutic agent for tauopathy, comprising the following steps (1) to (4):
   (1) contacting first neurons having a mutant MAPT gene with a test substance, followed by culture;
   (2) contacting the culture supernatant of the first neurons obtained in the step (1) with second neurons;
   (3) measuring any one index selected from the group consisting of the following (a) to (d) in the second neurons obtained in the step (2):
      (a) the amount of an intracellular tau oligomer,
      (b) the frequency of depolarization,
      (c) an intracellular calcium ion concentration, and
      (d) the number of living cells; and
   (4) selecting the test substance as a prophylactic or therapeutic agent for tauopathy when the value of (a), (b), or (c) measured in the step (3) after the contact of the first neurons with the test substance in the step (1) is lower than the value obtained without the contact of the first neurons with the test substance in the step (1), and/or when the value of (d) measured in the step (3) after the contact of the first neurons with the test substance in the step (1) is higher than the value obtained without the contact of the first neurons with the test substance in the step (1).
[2] A method for screening for a prophylactic or therapeutic agent for tauopathy, comprising the following steps (1) to (4):
   (1) contacting a culture supernatant of first neurons having a mutant MAPT gene with second neurons;
   (2) further contacting the second neurons with a test substance;
   (3) measuring any one index selected from the group consisting of the following (a) to (d) in the second neurons obtained in the step (2):
      (a) the amount of an intracellular tau oligomer,
      (b) the frequency of depolarization,
      (c) an intracellular calcium ion concentration, and
      (d) the number of living cells; and
   (4) selecting the test substance as a prophylactic or therapeutic agent for tauopathy when the value of (a), (b), or (c) measured in the step (3) after the contact of the second neurons with the test substance in the step (2) is lower than the value obtained without the contact of the second neurons with the test substance in the step (2), and/or when the value of (d) measured in the step (3) after the contact of the second neurons with the test substance in the step (2) is higher than the value obtained without the contact of the second neurons with the test substance in the step (2).
[3] The method according to [1] or [2], wherein the neurons or the first neurons having a mutant MAPT gene have been differentiated by the expression of foreign Neurogenin 2 from pluripotent stem cells having the mutant MAPT gene.
[4] The method according to any one of [1] to [3], wherein the second neurons have been differentiated by the expression of foreign Neurogenin 2 from pluripotent stem cells lacking a mutant MAPT gene.
[5] The method according to any one of [1] to [4], wherein the amount of an intracellular tau oligomer is the amount of the tau oligomer bound to lipid raft on the cells.
[6] The method according to any one of [1] to [5], wherein the neurons having a mutant MAPT gene express the mutant MAPT gene under the control of a nervous system-specific promoter.
[7] The method according to any one of [3] to [6], wherein the pluripotent stem cells having the mutant MAPT gene are induced pluripotent stem cells established from somatic cells of a tauopathy patient.
[8] The method according to any one of [1] to [7], wherein the mutant MAPT gene is a MAPT gene having one or more mutations at exons 9 to 13 or intron 10.
[9] The method according to [8], wherein the mutations at exons 9 to 13 are mutations to form mutant tau proteins each having one or more amino acid mutations selected from K257T, I260V, G272V, N297K, K280Δ, L284L, N296N, P301L, P301S, S305N, S305S, V337M, E342V, G389R, and R406W.
[10] The method according to [8], wherein the mutations at intron 10 are one or more mutations in nucleotides at positions 1 to 20 of the intron 10.
[11] The method according to [2], wherein the step (1) is the step of contacting a tau oligomer isolated from the culture supernatant of the first neurons having a mutant MAPT gene with the second neurons.

### EFFECT OF THE INVENTION

The present invention provides a method for screening for a prophylactic or therapeutic agent for tauopathy capable of blocking the propagation of tau-mediated neurodegeneration as defined by the claims.

### BRIEF DESCRIPTION OF THE DRAWINGS

In the description below, the number represented by n in a sentence represents the number of members in an experimental group (culture wells) subjected to the same operation. Results obtained in Examples of the present application were subjected to a significance test (JMP8 software) using student's-test or one-way ANOVA followed by Tukey post hoc analysis. In the case of p < 0.05, results were confirmed to be significant and indicated by asterisk (*) in drawings.

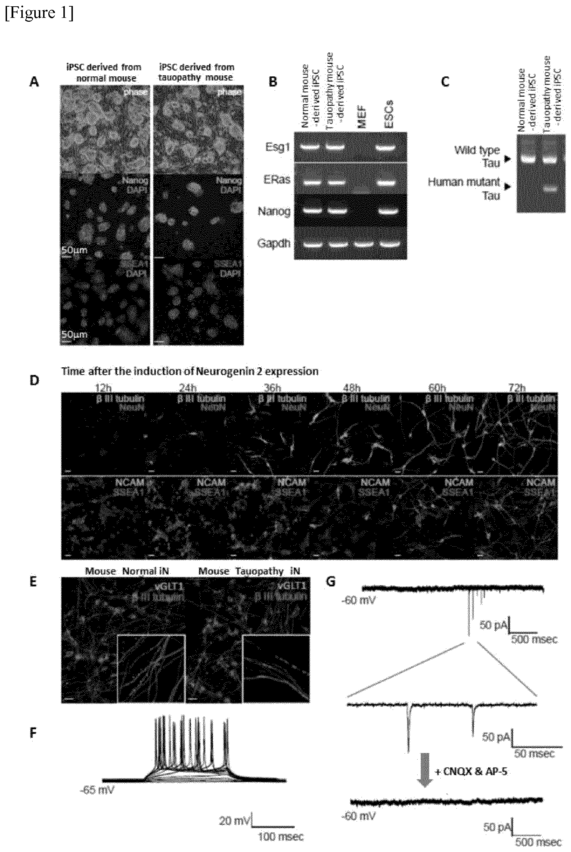

[Figure 1] Figures 1A and 1B show results of confirming, by immunostaining (Figure 1A) or RT-PCR (Figure 1B), that iPSCs prepared from a transgenic mouse harboring a P301S mutant human MAPT gene (tauopathy mouse), or its non-transgenic litter (normal mouse) express an undifferentiation marker (Esgl, Eras, Nanog, or SSEA1). In Figure 1B, tauopathy mouse MEFs (MEF) were used as a negative control, and mouse embryonic stem cells (ESCs) were used as a positive control. Figure 1C shows results indicating that the human mutant tau gene is slightly expressed in tauopathy mouse-derived iPSCs (RT-PCR). Figure 1D shows immunostaining photographs obtained by analyzing the expression of neuron markers (βIII-tubulin and NeuN) (upper photograph) and the expression of a neuron marker (N-CAM) and an undifferentiation marker (SSEA1) on cell surface (lower photograph) after 0 to 72 hours from the induction of foreign Neurogenin 2 expression in normal control mouse-derived iPSCs for neural differentiation. Figure 1E shows an immunostaining photograph indicating that mouse tauopathy iNs and mouse normal iNs express a glutamatergic neuron marker (vGLT1) and a neuron marker (βIII-tubulin). The inserted drawings show enlarged diagrams of neurites. Figure 1F shows action potential measured by a current clamp method in mouse normal iNs. Figure 1G shows results indicating that the generation of excitatory postsynaptic current is suppressed by the administration of CNQX and AP-5 to mouse normal iNs.

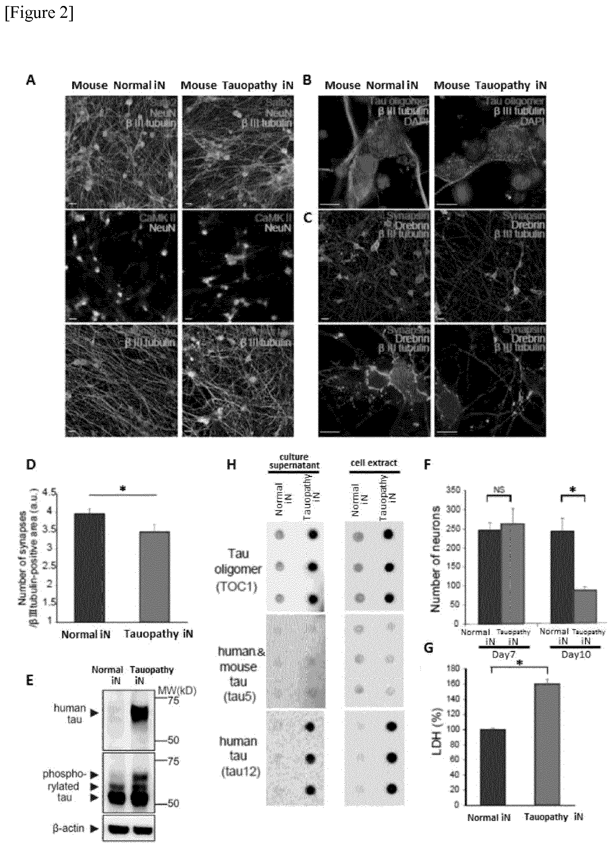

[Figure 2] Figure 2A shows immunostaining photographs indicating that mouse tauopathy iNs and mouse normal iNs of day 7 express neuron markers (NeuN and βIII-tubulin), a neuron marker (Satb2) of the layers II and III of the cerebral cortex, and a pyramidal cell marker (CaMKII), and the mouse tauopathy iNs further express human tau. Figure 2B shows photographs obtained by immunostaining for a tau oligomer and βIII-tubulin and nuclear staining (trichrome staining) with DAPI in mouse tauopathy iNs and mouse normal iNs of day 10. Figures 2C and 2D show results of visualizing synapse sites by double immunostaining for a presynaptic marker (Synapsin 1) and a postsynaptic marker (Drebrin) (Figure 2C) and measuring the number of the synapses (Figure 2D) in mouse tauopathy iNs and mouse normal iNs. The ordinate of Figure 2D depicts the number of synapses (= the number of dots costained with Synapsin 1 and Drebrin) per neuron (= βIII-tubulin-positive cell). Figure 2E shows results of conducting the Western blot of cell lysates prepared from mouse tauopathy iNs or mouse normal iNs of day 8. Figure 2F shows results of measuring the number of living cells as to mouse normal iNs and tauopathy iNs of days 7 and 10 (n = 6). In Figure 2G, culture supernatants of mouse normal iNs or tauopathy iNs of days 7 and 10 were prepared, and lactate dehydrogenase (LDH) activity in the culture supernatant of day 10 was indicated by relative value to the activity in the culture supernatant of day 7 (n = 6). Figure 2H shows results of preparing a culture supernatant and cell extracts from the culture system of mouse tauopathy iNs or mouse normal iNs of day 8 and analyzing the amount of a tau oligomer (TOC1), the amount of human tau (Tau 12), and the total amount of tau (Tau 5) by use of dot blot.

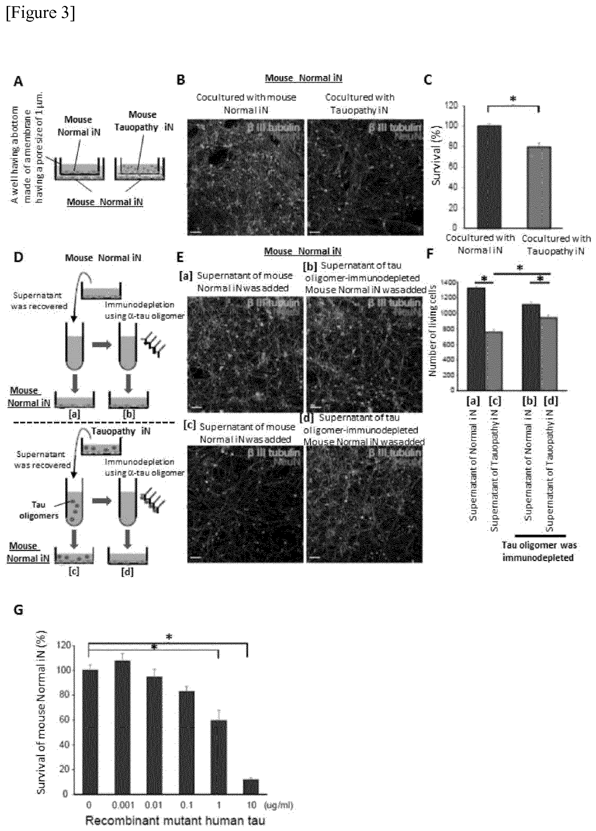

[Figure 3] Figure 3A shows schematic diagrams of the coculture system of mouse tauopathy iNs and mouse normal iNs (left diagram) and the coculture system of mouse normal iNs and mouse normal iNs as a control thereof (right diagram). Figures 3B and 3C show an immunostaining photograph of mouse normal iNs cocultured with mouse tauopathy iNs or mouse normal iNs for 7 days, using neuron markers (βIII-tubulin and NeuN) (Figure 3B), and change in the number of live neurons for these 7 days (Figure 3C; the number of living cells at the start day of the coculture was defined as 100%, n = 3). Figure 3D shows schematic diagrams of culture systems of adding a culture supernatant of mouse normal iNs (diagrams above the broken line) or a culture supernatant of mouse tauopathy iNs (diagrams below the broken line) to a medium of mouse normal iNs. Each culture system includes a system of directly adding the culture supernatant to the medium of mouse normal iNs (left diagrams) and a system of subjecting the culture supernatant to immunodepletion using an anti-tau oligomer antibody and then adding the resulting culture supernatant to the medium of mouse normal iNs (right diagrams). Figures 3E and 3F show photographs obtained by adding each culture supernatant shown in Figure 3D to mouse normal iNs of day 8 and immunostaining the cells with neuron markers (βIII-tubulin and NeuN) at day 10 (Figure 3E), and results of measuring a survival rate (= (the number of neurons of day 10/the number of neurons of day 8) × 100) (Figure 3F, n = 6).

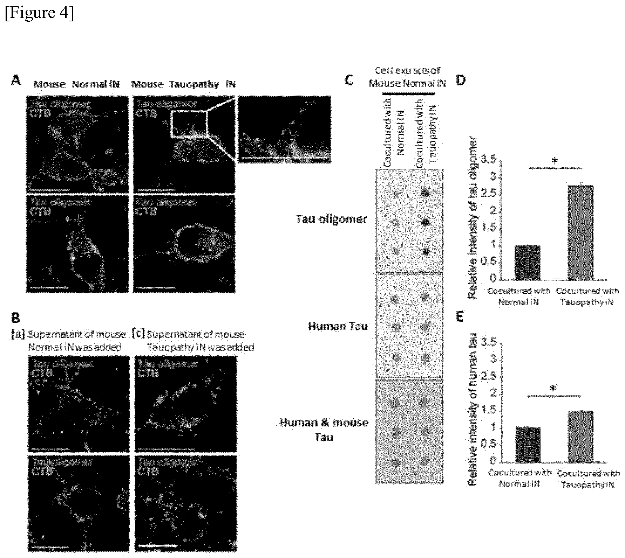

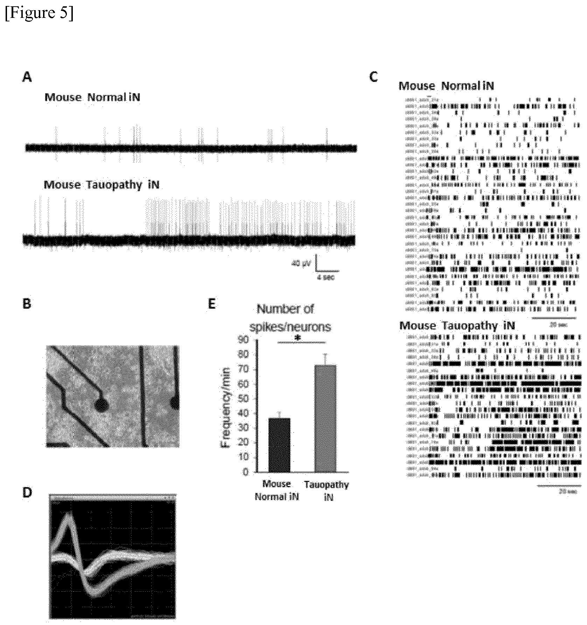

[Figure 4] Figure 4A shows results of subjecting mouse tauopathy iNs and mouse normal iNs of day 8 to CTB staining (which indicates lipid raft sites) and immunostaining (double staining) with an anti-tau oligomer antibody. Figure 4B shows results of adding a culture supernatant of mouse tauopathy iNs or mouse normal iNs to a medium of mouse normal iNs of day 8, followed by CTB staining/tau oligomer immunostaining (double staining) at day 10. Figures 4C to 4E show results of preparing cell extracts from mouse normal iNs cocultured with mouse tauopathy iNs or mouse normal iNs for 5 days, and analyzing the amount of a tau oligomer, the amount of human tau, and the total amount of tau in the extracts by dot blot (Figure 4C), and quantifying tau oligomer signals and human tau signals (Figures 4D and 4E). [Figure 5] Figure 5 shows results of measuring the spontaneous firing rate of mouse tauopathy iNs or mouse normal iNs of day 8 using a MEA system. Figure 5A shows the measured spontaneous firing rate. Figure 5B shows a photograph of iNs cultured on an electrode dish. Figure 5C shows spikes sorted to individual units. Figure 5D shows the waveforms of the sorted spikes. Figure 5E shows a spontaneous firing rate per neuron calculated from the results of Figures A and C.

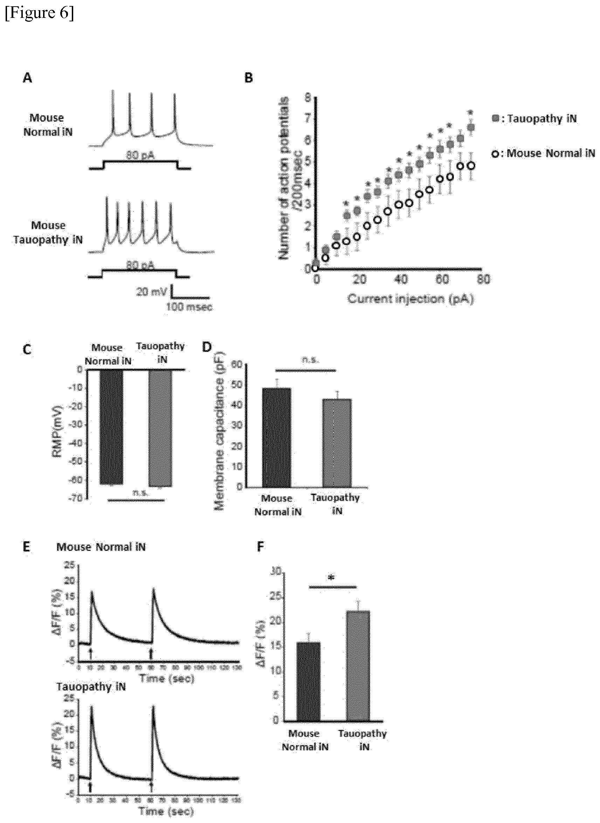

[Figure 6] Figures 6A to 6D show results of analyzing mouse tauopathy iNs and mouse normal iNs of day 7 by a whole-cell patch clamp method. These figures show results of measuring a firing rate after current injection (Figures 6A and B), resting membrane potential (Figure 6C), and membrane capacitance (Figure 6D). Figures 6E and 6F show results of measuring change in intracellular calcium concentration between before and after electric stimulation by use of a calcium imaging method in mouse tauopathy iNs or mouse normal iNs of day 7 (Figure 6E, n = 6). The arrows shown in the abscissa represent time points at which the cells were given electric stimulation. The ordinate (ΔF/F) depicts change in the fluorescence intensity of a calcium indicator (Fluo-8/AM) after the stimulation with respect to its baseline fluorescence intensity. Figure 6F is a graph on which the largest value of the ΔF/F was plotted.

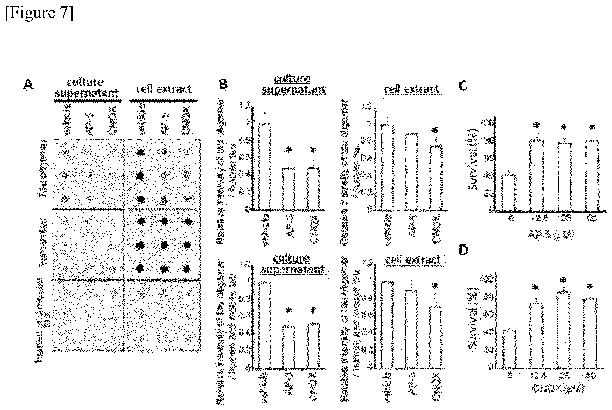

[Figure 7] Figures 7A and 7B show results of adding AP-5 (final concentration: 25 µM) or CNQX (final concentration: 25 µM) to mouse tauopathy iNs or mouse normal iNs of day 5, preparing a culture supernatant and cell extracts 48 hours later, and analyzing the amounts of tau and a tau oligomer contained in the culture supernatant and the cell extracts by dot blot (Figure 7A), and the results of quantifying their signals (Figure 7B). In Figures 7C and 7D, AP-5 or CNQX was added to mouse tauopathy iNs of day 7, and a survival rate at day 10 (= the number of live neurons at day 10/the number of live neurons at day 7) × 100) was indicated in a graph.

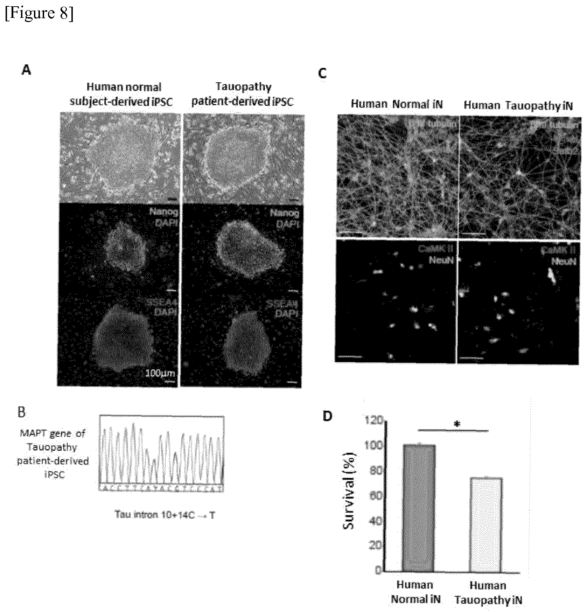

[Figure 8] Figure 8A shows phase contrast microscope photographs (upper photographs) and immunostaining photographs obtained by analyzing the expression of undifferentiated cell markers (middle photographs: Nanog, lower photographs: SSEA4), as to iPSCs prepared from fibroblasts of a FTDP-17 patient having an intron 10 mutant (intron 10 +14C > T) MAPT gene (right panels) and a human normal control-derived iPSC line (left panels). Figure 8B shows sequencing results indicating that the FTDP-17 patient-derived iPSCs retain the mutant MAPT gene. Figure 8C shows immunostaining photographs indicating that human tauopathy iNs and human normal iNs of day 10 express a neuron marker (Satb2) of the layers II and III of the cerebral cortex, neuron markers (NeuN and βIII-tubulin), and a pyramidal cell marker (CaMKII). Figure 8D is a graph indicating the number of live iNs of each type of day 20 as a ratio to the number of living cells at day 8 (n = 6).

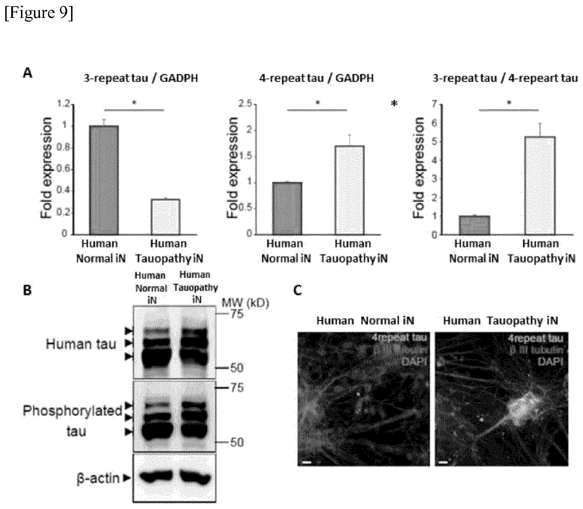

[Figure 9] Figure 9A shows results of analyzing the expression levels of 3-repeat tau and 4-repeat tau by use of RT-PCR in human tauopathy iNs and human normal iNs of day 14 and calculating a 4-repeat tau/3-repeat tau expression ratio. Figure 9B shows results of analyzing the expression levels of tau and phosphorylated tau by use of Western blot in human tauopathy iNs and human normal iNs of day 14. Figure 9C shows photographs obtained by subjecting human tauopathy iNs and human normal iNs to immunostaining with 4-repeat tau and βIII-tubulin and nuclear staining (trichrome staining) with DAPI.

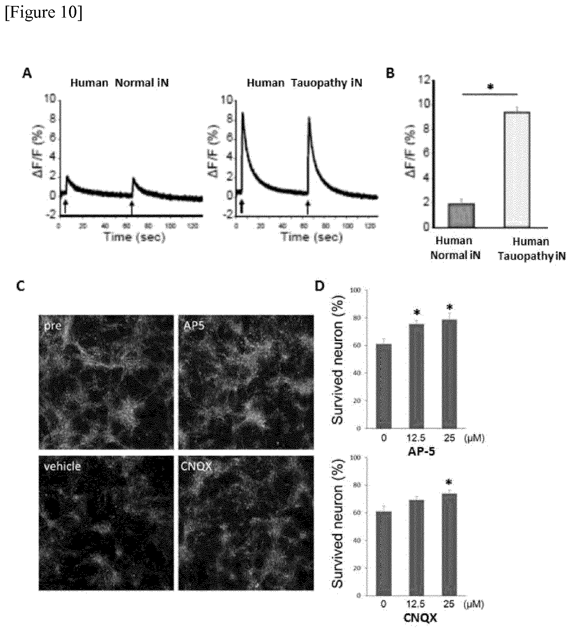

[Figure 10] Figures 10A and 10B show results of measuring change in intracellular calcium concentration between before and after electric stimulation by use of a calcium imaging method in human tauopathy iNs or human normal iNs of day 12 (Figure 10A, n = 6). The arrows shown in the abscissa represent time points at which the cells were given electric stimulation. The ordinate (ΔF/F) depicts change in the fluorescence intensity of a calcium indicator (Fluo-8/AM) after the stimulation with respect to its baseline fluorescence intensity.
Figure 10B is a graph on which the largest value of the ΔF/F was plotted. Figure 10C shows photographs obtained by adding AP-5 or CNQX (final concentration: 25 µM) to human tauopathy iNs of day 16, followed by double immunostaining with antibodies against neuron markers (βIII-tubulin and NeuN) at day 21. The upper left panel "pre" depicts the human tauopathy iNs of day 16 before the addition of the reagent. Figure 10D shows results (graphs) of analyzing a cell survival rate at day 21 (= (the number of live neurons at day 21/the number of live neurons at day 16) × 100) when the concentrations of AP-5 and CNQX in Figure 10C were changed.

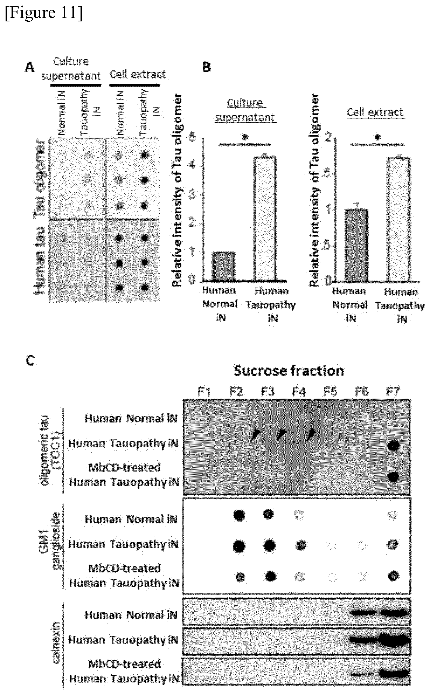

[Figure 11] Figures 11A and 11B show results of preparing a culture supernatant and cell extracts from human tauopathy iNs or human normal iNs of day 14 and analyzing the amounts of human tau and a tau oligomer contained in the culture supernatant or the cell extracts by use of dot blot (Figure 11A), and graphs on which their signals were quantified (Figure 11B). Figure 11C shows results of fractionating cell homogenates prepared from human normal iNs, human tauopathy iNs, or MbCD-treated human tauopathy iNs by use of a sucrose density-gradient centrifugation method, and analyzing a tau oligomer (upper diagram: Western blot) and a lipid raft component (middle diagram: CTB staining) contained in the obtained fractions (F1 to F7). The lower diagram shows Western blot results of an endoplasmic reticulum marker Calnexin.

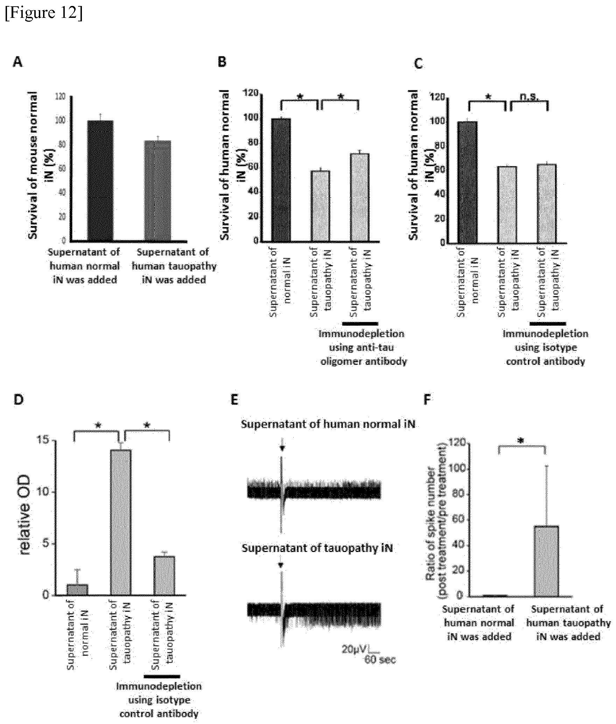

[Figure 12] Figure 12A shows results of adding a culture supernatant of human tauopathy iNs or human normal iNs to a medium of mouse normal iNs of day 8 and measuring a cell survival rate 2 days later (n = 6). Figures 12B and 12C show results of subjecting a culture supernatant of human tauopathy iNs to immunodepletion using an anti-tau oligomer antibody (Figure 12B) or an isotype control antibody thereof (Figure 12C), then adding the resulting culture supernatant to a medium of human normal iNs of day 16, and measuring a cell survival rate 2 days later (= (the number of neurons of day 18/the number of neurons of day 16) × 100) (n = 6). Figure 12D shows results of measuring the amount (concentration) of a tau oligomer in each culture supernatant used in Figure 12C by ELISA. Figure 12E shows results of adding a culture supernatant of human normal iNs or human tauopathy iNs to human normal iNs of day 20 and measuring a spontaneous firing rate using a MEA system. The arrows represent the timing at which each culture supernatant was added. Figure 12F is a graph indicating the ratio of spike number between before and after the addition measured in Figure 12E.

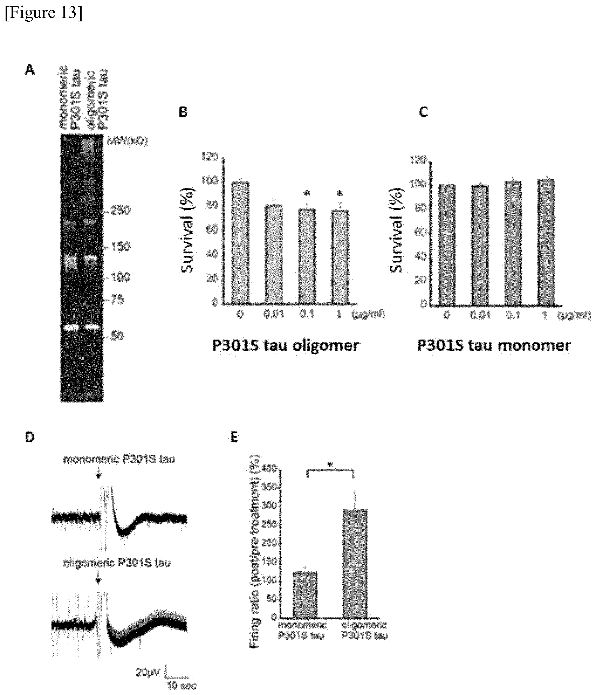

[Figure 13] Figure 12A shows results of subjecting recombinant P301S mutant human tau (left lane: tau monomer) and the mutant tau treated with heparin *in vitro* (right lane: tau oligomer) to SDS-PAGE followed by silver staining. Figure 13B shows results of adding the tau monomer (Figure 13B) or the tau oligomer (Figure 13C) of Figure 13A to a medium of human normal iNs of day 16 and measuring a cell survival rate 2 days later (= (the number of neurons of day 18/the number of neurons of day 16) × 100). Figures 13D and 13F show results of adding the tau monomer or the tau oligomer of Figure 13A to a medium of human normal iNs of day 20 and measuring a spontaneous firing rate using a MEA system (Figure 13D). The arrows represent the timing at which the tau monomer or the tau oligomer was added. Figure 13E is a graph indicating the ratio of spike number between before and after the addition.

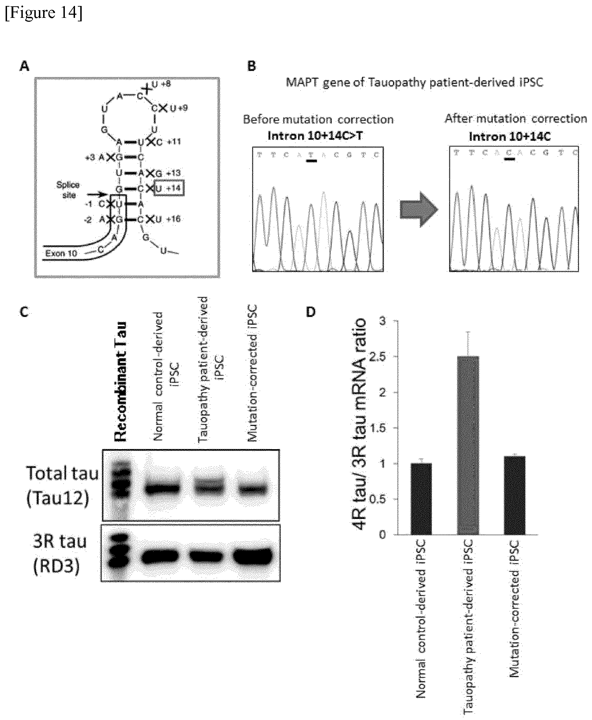

[Figure 14] Figure 14A is a diagram illustrating a mutation (intron 10 +14C > T) as to mRNA transcribed from a mutant MAPT gene of a tauopathy patient. In mRNA having a putative secondary structure, the mutation site (uracil at position 14 of intron 10) is boxed. Figure 14B shows results of sequencing the mutant MAPT gene in tauopathy patient iPSCs (left diagram: before mutation correction) and a MAPT gene in which the mutation was corrected to wild type in iPSCs (right diagram: after correction correction). Figure 14C shows results of subjecting normal control-derived iPSCs, tauopathy patient-derived iPSCs, and mutation-corrected iPSCs to wstern blot and analyzing the total amount of tau (Tau 12) and the amount of 3-repeat tau (RD3). Figure 14D shows results of analyzing a 4-repeat tau/3-repeat tau expression ratio by use of RT-PCR in normal control-derived iPSCs, tauopathy patient-derived iPSCs, and mutation-corrected iPSCs.

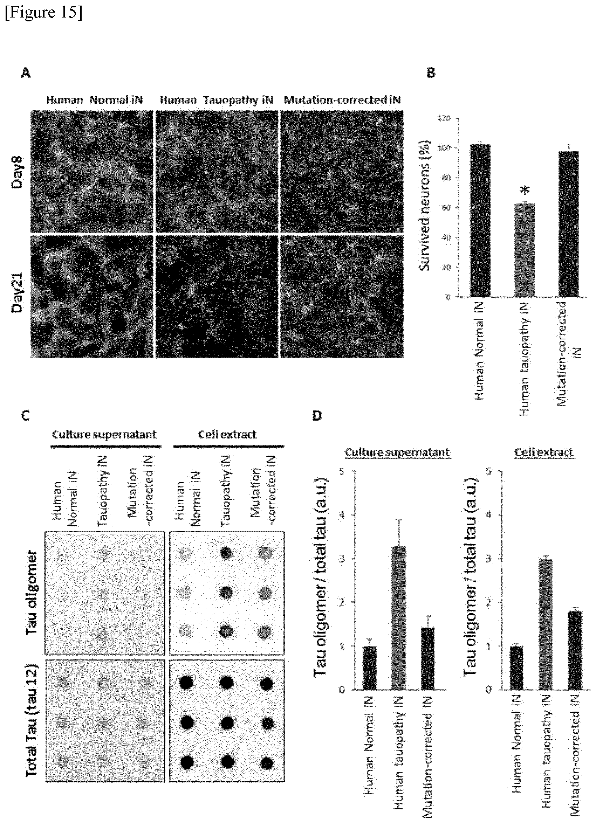

[Figure 15] Figures 15A and 15B show photographs obtained by double-immunostaining human normal iNs, human tauopathy iNs, and mutation-corrected iNs of day 8 (upper photographs) and day 21 (lower photographs) using antibodies against neuron markers (βIII-tubulin and NeuN) (Figure 15A), and a graph on which a survival rate (= (the number of live neurons at day 21/the number of live neurons at day 8) × 100) was calculated (Figure 15B). Figure 15C shows results of preparing a culture supernatant and cell extracts from the culture system of human normal iNs, human tauopathy iNs, or mutation-corrected iNs of day 14 and analyzing the amount of a tau oligomer and the total amount of tau (Tau 12) by use of dot blot. Figure 15D is a graph on which the ratio of a tau oligomer to the total amount of tau (= the amount of a tau oligomer/the total amount of tau) was calculated by quantifying the signals obtained in Figure 15C.

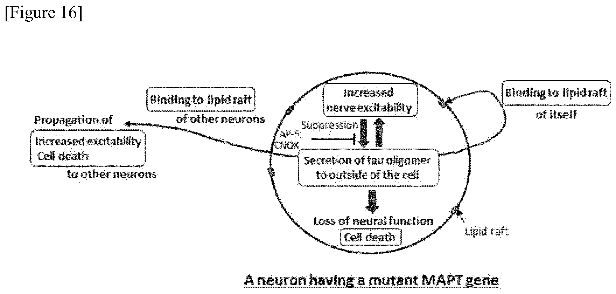

[Figure 16] Figure 16 is a diagram showing the pathological conditions of tauopathy exhibited by neurons having a mutant MAPT gene. The boxed pathological conditions represent indexes used in the screening method according to the present invention.

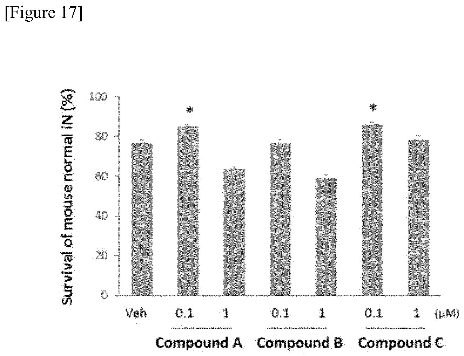

[Figure 17] Figure 17 shows results of performing screening by using a cell death inhibitory effect on normal iNs as an index in a culture system in which a culture supernatant of tauopathy iNs was added to the normal iNs.

### BEST MODE FOR CARRYING OUT THE INVENTION

Hereinafter, preferred embodiments of the present invention will be described in detail.

In the present specification, DNA having a sequence encoding a particular protein and control sequences necessary for the expression of the protein (e.g., a promoter, an enhancer, a ribosomal binding sequence, a terminator, and a polyadenylation site) is referred to as a "gene". In the present specification, "NP_", "NM_", or "NM_" and the subsequent numeral represent ID of an amino acid sequence (NP_XX), a transcript nucleotide sequence (NM_XX), or a genomic DNA sequence (NM_XX) registered as a reference sequence in the NCBI (National Center for Biotechnology Information) database.

In the present invention, the normal neurons refer to neurons having no MAPT gene mutation. In the present specification, numeric values connected via a hyphen may represent a numeric range including the numeric values (e.g., the term "3-5 days" means "3 days or longer and 5 days or shorter").

### <Tau>

Microtubule-associated protein tau (also called MAPT) is a protein encoded by MAPT gene (official full name: microtubule-associated protein tau, official symbol; MAPT, NG_007398.1) that resides on chromosome 17 (17q21.1) in humans. Six isoforms formed by alternative splicing have been identified. These isoforms differ in the number of N-terminal characteristic 29-amino acid sequences (N) (0 to 2) and the number of C-terminal repeat sequences (R) involved in microtubule binding (3 or 4) and are therefore classified, depending on the numbers of these sequences, into 0N3R tau (352 amino acids, NP_058525.1, NM_016841.4), 1N3R tau (381 amino acids, NP_001190180.1, NM_001203251.1), 2N3R tau (410 amino acids, NP_001190181.1, NM_001203252.1), 0N4R tau (383 amino acids, NP_058518.1, NM_016834.4), 1N4R tau (412 amino acids, NP_001116539.1, NM_001123067.3), and 2N4R tau (441 amino acids, NP_005901.2, NM_005910.5) (the number of amino acid residues in each human isoform, ID of a reference amino acid sequence, and ID of a reference nucleotide sequence of a transcript are shown within the parentheses). All of the 6 types are expressed in the human adult brain. In normal humans, an expression ratio between 4R tau (4-repeat tau) and 3R tau (3-repeat tau) (= 4-repeat tau/3-repeat tau) is almost 1:1 (Non-Patent Documents 1, 6, and 8). In the present invention, the tau means 3-repeat tau and 4-repeat tau without any distinction, unless otherwise specified.

### <Mutant MAPT gene>

In the present invention, mutant MAPT genes identified from FTDP-17 families can be preferably used. (i) Mutations to change the amino acid sequence of the tau protein and (ii) mutations to elevate a 4-repeat tau/3-repeat tau expression ratio are known as the mutations. Any of the mutations can be preferably used in the present invention. The mutations (i) are gene mutations to form mutant tau proteins, and the mutations (ii) are gene mutations to form no mutant tau protein.

A mutation of the type (i) is indicated by amino acid mutation that occurs in the tau protein on the basis of the amino acid sequence of the longest isoform 2N4R tau (441 amino acids, NP_005901.2 (SEQ ID NO: 1), NM_005910.5 (SEQ ID NO: 2)). For example, the term "P301S" means a gene mutation to form a tau protein by the substitution of a proline residue (P) at position 301 of the amino acid sequence of NM_005910.5 by a serine residue (S), and the term "K280Δ" means a gene mutation to form a tau protein by the deletion of a lysine residue at position 280 of the sequence (i.e., a gene having this mutation encodes the mutant tau protein). Mutation sites of type (i) identified so far from FTDP-17 families are present mainly at exons 9 to 13. Therefore, in the present invention, a MAPT gene having mutation(s) at exons 9 to 13 can be particularly preferably used. Examples of the mutation(s) at exons 9 to 13 include one or more mutations selected from K257T, I260V, G272V, N297K, K280Δ, L284L, N296N, P301L, P301S, S305N, S305S, V337M, E342V, G389R, and R406W. Particularly, one or more mutations selected from G272V, N297K, P301L, P301S, V337M, and R406W are preferred. This is because prepared tauopathy mouse models harbor a mutant MAPT gene having one or more mutations selected from G272V, N297K, P301L, P301S, V337M, and R406W.

A large number of mutations within intron 10 have been identified as mutations of the type (ii) from FTDP-17 families. Their mutant MAPT genes can be preferably used. Examples of the nucleotide sequence of intron 10 in the wild-type MAPT gene include a nucleotide sequence consisting of bases at positions 120983 to 124833 of NG_007398.1 (SEQ ID NO: 3). Among others, a MAPT gene having one or more mutations in stem-loop formed by splicing and its neighborhood, i.e., nucleotides at positions 1 to 20 of intron 10 (SEQ ID NO: 3) is preferred. Examples of such a MAPT gene include a MAPT gene having a substitution in base at position 3, 11, 12, 13, 14, 16, or 19 (Non-Patent Document 6). Among these substitutions, the base substitution at position 3, 11, 13, 14, or 16 has been confirmed by an exon trapping method to inhibit the splicing of exon 10 and elevate a 4-repeat tau/3-repeat tau expression ratio (Non-Patent Document 7) and can be most preferably used in the present invention. Examples of the base substitution include intron 10 +3G > A, intron 10 +11T> C, intron 10 +13 A >G, intron 10 +14C > T, and intron 10 +16C > T. The term "intron 10 +3G > A" represents a mutation that substitutes a base guanine (G) at position 3 of intron 10 by adenine (A).

In the present invention, the mutation to elevate a 4-repeat tau/3-repeat tau expression ratio refers to a mutation that results in the expression ratio (which can be any of a mRNA level ratio and a protein level ratio and is preferably a mRNA level ratio) of 1.3 or more, preferably 1.5 or more. The mRNA level ratio is usually less than 1.3 in normal humans, whereas FTDP-17 patients having a mRNA level ratio of approximately 1.6 have been reported (Non-Patent Document 8). The expression ratio may be analyzed by expressing the mutant MAPT gene in appropriate cultured cells, and subjecting the cells to real-time PCR, RT-PCR, or Northern hybrydization or the like to determine the mRNA level of each isoform, or subjecting the cells to Western blot or the like to determine the protein level of each isoform. Alternatively, the expression ratio may be analyzed by an exon trapping method illustrated in Non-Patent Document 7.

In the present invention, neurons having a MAPT gene having the mutation(s) (i) to change the amino acid sequence of the tau protein and/or the mutation(s) (ii) to elevate a 4-repeat tau/3-repeat tau expression ratio can be preferably used as neurons having a mutant MAPT gene. The neurons may be neurons differentiated by induction from pluripotent stem cells having the mutant MAPT gene. Examples of the pluripotent stem cells having the mutant MAPT gene include pluripotent stem cells prepared from somatic cells of an animal endogenously carrying the mutant MAPT gene, pluripotent stem cells prepared from animal cells transfected with the mutant MAPT gene as a foreign gene, and pluripotent stem cells transfected with the mutant MAPT gene as a foreign gene.

Examples of the pluripotent stem cells prepared from somatic cells of an animal endogenously carrying the mutant MAPT gene include pluripotent stem cells prepared from somatic cells of a FTDP-17 patient or a transgenic mouse harboring the human mutant MAPT gene. The pluripotent stem cells are particularly preferably pluripotent stem cells prepared from somatic cells of a FTDP-17 patient.

The transfection with the mutant MAPT gene as a foreign gene means that cells are transfected with the mutant MAPT gene in a state containing control sequences such as a promoter, an enhancer, a ribosomal binding sequence, a terminator, and a polyadenylation site so as to be able to express a protein encoded by the gene. If necessary, the cells may be transfected with the mutant MAPT gene in a state further containing a drug resistance gene (e.g., kanamycin resistance gene, ampicillin resistance gene, or puromycin resistance gene), a selective marker sequence such as thymidine kinase gene or diphtheria toxin gene, or a gene sequence of a reporter such as green fluorescent protein (GFP), β glucuronidase (GUS), or FLAG.

In the present invention, for the mutant MAPT gene, it is preferred that its expression should be promoted or induced during the course of differentiation into neurons. Therefore, a nervous system-specific promoter is preferred as the promoter for the expression of the foreign MAPT gene. Examples of the nervous system-specific promoter include prion promoter (Neuroimage, 54: 2603-11, 2011), nestin promoter (Curr. Biol., 6: 1307-16, 1996), CaM kinase II promoter (Neuroimage, 54: 2603-11, 2011), PDGF-β promoter (Gene Thera., 11: 52-60, 2004), napsin 1 or neurofilament-H (NF-H) promoter (Nat. Cell Biol., 7: 474-82, 2005), and MAPT promoter (Japanese Patent No. 2008-61557).

### <Differentiation into neuron>

In the present invention, neurons differentiated from pluripotent stem cells can be preferably used as neurons.

Examples of the method for inducing the differentiation of pluripotent stem cells into neurons include, but are not particularly limited to, (1) a method which involves culturing the pluripotent stem cells in a serum-free medium to form embryoids (cell masses containing neural progenitor cells), which are differentiated into neurons (SFEB method: Watanabe K., et al., Nat. Neurosci., 8: 288-296, 2005; SFEBq method: Wataya T., et al., Proc. Natl. Acad. Sci. USA., 105: 11796-11801, 2008), (2) a method which involves differentiating the pluripotent stem cells into neurons by culture on stromal cells (SDIA method: Kawasaki H., et al., Neuron, 28: 31-40, 2000), (3) a method which involves differentiating the pluripotent stem cells into neurons by culture on Matrigel supplemented with a drug (Chambers S.M., et al., Nat. Biotechnol., 27: 275-280, 2009), (4) a method which involves differentiating the pluripotent stem cells into neurons by culture in a medium containing a low-molecular compound as a cytokine substitute (U.S. Patent No. 5,843,780), (5) a method which involves transfecting the pluripotent stem cells with a neural factor (neurogenin 2, etc.) and differentiating the pluripotent stem cells into neurons by the expression of this factor (Patent Document 7 and Non-Patent Document 10), and combinations of these methods (including modifications thereof).

Among these methods, the method (5) which involves transfecting the pluripotent stem cells with neurogenin 2 and differentiating the pluripotent stem cells into neurons by the expression of this factor produces mature neurons in a short period and with high efficiency and can therefore be particularly preferably used in the present invention. In the present specification, the neurogenin 2 refers to a gene, and the Neurogenin 2 refers to a protein encoded by the gene.

The Neurogenin 2 protein is a transcriptional factor known to promote differentiation into neurons at the stage of development. Examples of the amino acid sequence thereof include NP _076924 (SEQ ID NO: 4) in humans and NP_033848 in mice. In the present invention, the neurogenin 2 gene (official full name: neurogenin 2, official symbol: NEUROG2; also called Ngn2 gene) is DNA encoding the Neurogenin 2 protein. Examples thereof include DNA having the nucleotide sequence of NM_009718 (mouse) or NM_024019 (human) registered as a reference sequence, or a transcript variant thereof. Alternatively, the neurogenin 2 gene may be DNA having complementarity that permits hybridization under stringent conditions to a nucleic acid having the reference sequence or the sequence of the transcript variant.

The stringent conditions can be determined based on the melting temperature (Tm) of a complex or a probe-binding nucleic acid, as taught in Berger and Kimmel(1987, Guide to Molecular Cloning Techniques Methods in Enzymology, Vol.152, Academic Press, San Diego CA). For example, washing conditions after completion of the hybridization can generally be conditions such as "1xSSC, 0.1% SDS, and 37°C." A complementary strand preferably maintains a hybridized state with a plus strand as a target, even if it has been washed under the above described conditions. More stringent hybridization conditions can be conditions in which the plus strand and the complementary strand can maintain a hybridized state, even after completion of the washing under washing conditions such as "0.5xSSC, 0.1% SDS, and 42°C," and further stringent hybridization conditions can be conditions in which the plus strand and the complementary strand can maintain a hybridized state, even after completion of the washing under washing conditions such as "0.1xSSC, 0.1% SDS, and 65°C," although the conditions are not particularly limited thereto. Specifically, examples of such a complementary strand include a strand consisting of a nucleotide sequence having a completely complementary relationship with the nucleotide sequence of the target plus strand, and a strand consisting of a nucleotide sequence having an identity of at least 90%, preferably 95% or more, more preferably 97% or more, even more preferably 98% or more, and particularly preferably 99% or more with the above described strand.

In the present invention, an expression of Neurogenin2 in pluripotent stem cells can be carried out by introducing one or more nucleic acids (DNA or RNA) encoding Neurogenin2 or Neurogenin2 (Protein) into pluripotent stem cell.

In the present invention, when the nucleic acid encoding Neurogenin2 is in the form of DNA, it is introduced into a vector such as a virus, a plasmid or an artificial chromosome, and it can be then introduced into a pluripotent stem cell by a method such as lipofection, liposome, or microinjection. Examples of the virus vector include a retrovirus vector, a lentivirus vector, an adenovirus vector, an adeno-associated virus vector, and a Sendai virus vector. Examples of the artificial chromosome vector include a human artificial chromosome (HAC), a yeast artificial chromosome (YAC), and a bacterial artificial chromosome (BAC, PAC). As a plasmid vector, a plasmid for mammalian cells can be used. These vectors may comprise regulatory sequences such as a promoter, an enhancer, a ribosome binding sequence, a terminator and a polyadenylation site, so that Neurogenin2 protein can be expressed. Moreover, as necessary, these vectors may also comprise selection marker sequences such as drug resistance genes (e.g., a kanamycin resistance gene, an ampicillin resistance gene, a puromycin resistance gene, etc.), a thymidine kinase gene and a diphtheria toxin gene, and reporter gene sequences such as a fluorescent protein, β-glucuronidase (GUS) and FLAG. Specifically, the nucleic acids encoding amino acid sequence of Neurogenin2 may be operably linked to an inducible promoter, so that the expression of Neurogenin2 protein can be quickly induced at a desired time.

The inducible promoter can be a drug responsive promoter, and a preferred example of such a drug responsive promoter is a tetracycline-responsive promoter (a CMV minimal promoter having a tetracycline-responsive element (TRE) consisting of seven consecutive tetO sequences). Tet-On/Off Advanced expression inducing systems are exemplified. Tet-On system is preferable because this system enable an expression of a gene corresponding to the promoter in the presence of tetracycline. This system also requires an additional expression of a fusion protein composed of reverse tetR (rTetR) and VP16AD (rtTA). This system is available from Clontech Laboratories, Inc..

Moreover, a cumate-responsive promoter (Q-mate system, Krackeler Scientific, National Research Council (NRC), etc.), an estrogen-responsive promoter (WO2006/129735, and a GenoStat-inducible expression system, Upstate cell signaling solutions), and a RSL1-responsive promoter (a RheoSwitch mammal-inducible expression system, New England Biolabs) can be preferably used. Amon those systems, due to a high specificity of induction and a low toxicity, a tetracycline-responsive promoter and a cumate-responsive promoter are specifically preferable, and a tetracycline-responsive promoter is most preferable.

When a cumate-responsive promoter is used to induce the expression of the gene, it is more preferable to be accompanied with a system capable of expressing CymR repressor in a cell. regulatory sequences of promoter

The regulatory sequences and regulatory factors of the promoter (rtTA and/or CymR repressor, etc.) may be supplied from the vector in which neurogenin2 gene has been introduced.

When the tetracycline-responsive promoter is used, the expression of Neurogenin2 can be maintained by continuously adding tetracycline or DOX to a medium for a desired period of time. When cumate-responsive is used, the expression of Neurogenin2 can be maintained by continuously adding cumate to a medium for a desired period of time.

Thereafter, by removing the drug corresponding to the promoter from the medium (e.g., by replacing the medium with a medium that does not contain the drug), the expression of Neurogenin2 can be terminated.

In the present invention, when the nucleic acid encoding Neurogenin2 is in the form of RNA, it may be introduced into a pluripotent stem cell, for example, by a method such as electroporation, lipofection or microinjection. In order to maintain the expression of Neurogenin2 in the cell, the introduction may be carried out more than one, for example, twice, three times, four times, or five times.

In the present invention, when Neurogenin2 is introduced in the form of protein, it may be introduced into a pluripotent stem cell by a method, for example, lipofection, fusion with cell penetrating peptide (e.g., TAT derived from HIV and polyarginine), microinjection and the like. In order to maintain the expression of Neurogenin2 in the cell, the introduction may be carried out more than one, for example, twice, three times, four times, or five times.

In the present invention, the expression of the introduced Neurogenin2 for an induction of neurons is not limited because even if the expression is maintained for a long period of time, there are no disadvantages. The expression period is 3 days or more, preferably 4 days or more, and more preferably 5 days or more when a pluripotent stem cell is a mouse cell. The expression period is 6 days or more, preferably 7 days or more, and more preferably 8 days or more when a pluripotent stem cell is a human cell.

It is preferred that, after the induction of Neurogenin2 expression, the pluripotent stem cells is cultured in a medium suitable for inducing differentiation into neurons (hereinafter, referred to as a media for neural differentiation).

As such a medium, a basal medium or a basal medium supplemented with neurotrophic factors can be used. In the present invention, a neurotrophic factor is a ligand of a membrane receptor which plays a crucial role in maintaining survival and function of motor neurons. For example, Nerve Growth Factor (NGF), Brain-derived Neurotrophic Factor (BDNF) , Neurotrophin 3 (NT-3), Neurotrophin 4/5 (NT-4/5), Neurotrophin 6 (NT-6), basic FGF, acidic FGF, FGF-5, Epidermal Growth Factor (EGF), Hepatocyte Growth Factor (HGF), Insulin, Insulin Like Growth Factor 1 (IGF 1), Insulin Like Growth Factor 2 (IGF 2), Glia cell line-derived Neurotrophic Factor (GDNF), TGF-b2, TGF-b3, Interleukin 6 (IL-6), like Ciliary Neurotrophic Factor (CNTF), and LIF, etc. are mentioned. A preferred neurotrophic factor in the present invention is a factor selected from the group consisting of GDNF, and BDNF. Examples of the basal medium include Glasgow's Minimal Essential Medium (GMEM), IMDM, Medium 199, Eagle's Minimum Essential Medium (EMEM), αMEM, Dulbecco's modified Eagle's Medium (DMEM), Ham's F12 medium, RPMI 1640 medium, Fischer's medium, Neurobasal Medium (Life Technologies), and the mixtures thereof, etc. The basal medium may contain serum or it may be serum-free. If necessary, the medium may contain one or more serum replacement such as, for example, Knockout Serum Replacement (KSR) (serum substitute for FBS in ES cell culture), N2 supplement (Invitrogen), B27 supplement (Invitrogen), albumin, transferrin, apotransferrin, fatty acids, insulin, collagen precursor, trace elements, 2-mercaptoethanol, and 3'-thiol glycerol. The medium may also contain one or more substances such as, for example, lipids, amino acids, L- glutamine, Glutamax (Invitrogen), nonessential amino acids, vitamins, growth factors, small molecule compounds, antibiotics, antioxidants, pyruvic acid, buffering agents, inorganic salts, selenate, progesterone, and putrescine, if necessary.

In the present invention, as a media for neural differentiation, a mixture of DMEM/F12 medium and Neurobasal Medium (the mixing volume ratio of 1:1) supplemented with N2 supplement, B27 supplement, BDNF, GDNF, and NT3 can be preferably used.

The culturing temperature for inducing differentiation into neurons is not particularly limited, and about 30 to 40°C, preferably about 37°C, in an air atmosphere containing CO₂. The CO₂ concentration is preferably about 5%.

In the present invention, a neuron is defined as a cell that expresses at least one or more marker genes specific to neurons selected from β-III tubulin, NCAM (neural cell adhesion molecule), and MAP2 (microtubule-associated protein 2), and has one or more β-III tubulin-positive processes (hereinafter, referred to as neurites).

In the present invention, the neurons are more preferably morphologically mature neurons, further preferably glutamatergic neurons. In this context, the morphologically mature neurons mean neurons having a thickened cell body and sufficiently extended neurites (as a guideline, the neurite length is approximately 5 times or more the diameter of the cell body). As shown in Examples of the present application, for example, in the case of inducing the differentiation of mouse pluripotent stem cells into neurons by the induction of foreign Neurogenin 2 expression, morphologically mature glutamatergic neurons can be obtained approximately 3 days after the induction of expression. When the pluripotent stem cells are human pluripotent stem cells, morphologically mature glutamatergic neurons can be obtained approximately 10 days after the induction of Neurogenin 2 expression.

### < Pluripotent stem cells >

In the present invention, the pluripotent stem cell means a stem cell that has a pluripotency of differentiating into all types of cells present in a living body and also has a proliferating ability. Examples of the pluripotent stem cells include, but are not limited to, embryonic stem (ES) cells, non-human nuclear transfer embryonic stem (ntES) cells derived from a cloned embryo obtained by nuclear transfer, spermatogonial stem cells ("GS cells"), embryonic germ cells ("EG cells"), induced pluripotent stem (iPS) cells, and pluripotent cells derived from cultured fibroblasts or bone marrow stem cells (Muse cells). Among these cells, pluripotent stem cells preferably used in the present invention include ES cells, non-human ntES cells, and iPS cells. Hereinafter, each of these stem cells will be described.

### (A) Embryonic stem cells

ES cell is a stem cell having a pluripotency and a proliferating ability associated with self-replication, which can be established from an inner cell mass of an early embryo (e.g., a blastocyst) of a mammal such as a human or a mouse.

The ES cell is a stem cell which can be derived from an embryo from the inner cells mass of a blastocyst that is an embryo after the 8-cell stage and morula stage of a fertilized egg, and this cell has an ability to differentiate into all types of cells constituting an adult body, namely, differentiation pluripotency, and proliferating ability associated with self-replication. The ES cells were discovered in a mouse in 1981 (M.J. Evans and M.H. Kaufman (1981), Nature 292:154-156), and thereafter, the ES cell line was also established in primates such as a human and a monkey (J.A. Thomson et al. (1998), Science 282:1145-1147; J.A. Thomson et al. (1995), Proc. Natl. Acad. Sci. USA, 92:7844-7848;J.A. Thomson et al. (1996), Biol. Reprod., 55:254-259; J.A. Thomson and V.S. Marshall (1998), Curr. Top. Dev. Biol., 38:133-165).

The ES cells can be established by extracting an inner cell mass from the blastocyst of the fertilized egg of a target animal and then culturing the inner cell mass on a fibroblast feeder. In addition, retention of the cells by subculture can be carried out using a culture, to which a substance such as a leukemia inhibitory factor (LIF) or a basic fibroblast growth factor (bFGF) has been added. Methods for establishing and retaining the ES cells of a human and a monkey are described, for example, in USP5,843,780; Thomson JA, et al. (1995), Proc Natl. Acad. Sci. U S A. 92:7844-7848 ; Thomson JA, et al. (1998), Science. 282:1145-1147 ; H. Suemori et al. (2006), Biochem. Biophys. Res. Commun., 345:926-932 ; M. Ueno et al. (2006), Proc. Natl. Acad. Sci. USA, 103:9554-9559; H. Suemori et al. (2001), Dev. Dyn., 222:273-279; H. Kawasaki et al. (2002), Proc. Natl. Acad. Sci. USA, 99:1580-1585 ; Klimanskaya I, et al. (2006), Nature. 444:481-485; etc.

As a culture used to produce ES cells, for example, a DMEM/F-12 culture, to which 0.1 mM 2-mercaptoethanol, 0.1 mM non-essential amino acid, 2 mM L-glutamic acid, 20% KSR and 4ng/ml bFGF have been replenished, is used, and human ES cells can be retained at 37°C in a wet atmosphere of 2% CO₂/98% air (O. Fumitaka et al. (2008), Nat. Biotechnol., 26:215-224). Moreover, the ES cells need to be subcultured every 3 or 4 days, and the subculture can be carried out, for example, using 0.25% trypsin and 0.1 mg/ml collagenase IV in PBS that contains 1 mM CaCl₂ and 20% KSR.

The ES cells can be generally selected by a Real-Time PCR method, using the expression of a gene marker such as alkaline phosphatase, Oct-3/4 or Nanog, as an indicator. In particular, when human ES cells are selected, the expression of a gene marker specific to undifferentiated cells, such as OCT-3/4, NANOG, or ECAD, can be used as an indicator (E. Kroon et al. (2008), Nat. Biotechnol., 26:443-452).

Reference human ES cell lines, for example WA01 (HI) and WA09 (H9) are available from WiCell Reserch Institute, and KhES-1, KhES-2 and KhES-3 are available from the Kyoto University Institute for Frontier Medical Sciences (Kyoto, Japan).

### (B) Spermatogonial stem cells

Spermatogonial stem cell is a pluripotent stem cell derived from testis, which serves as an origin for formation of the sperm. The cell can be induced to differentiate into cells of various lines, as well as ES cells, and has properties such that if the cell is transplanted, for example, in a mouse blastocyst, a chimeric mouse can be produced (M. Kanatsu-Shinohara et al, Biol Reprod, 69:.. 612-616, 2003; K. Shinohara et al, Cell, 119: 1001-1012, 2004). The spermatogonial stem cells are able to self-replicate in a culture medium containing glial cell line-derived neurotrophic factor (GDNF), and such spermatogonial stem cells can also be obtained by repeating subculture under the same culture conditions as those of ES cells cells (Takebayashi Masanori et al. (2008), Experimental Medicine, Vol. 26, No. 5 (special edition), 41-46 pages, Yodosha (Tokyo, Japan), 2008).

### (C) Embryonic germ cells

Embryonic germ cell is established from a primordial germ cell at embryonic stage and has a pluripotency similar to ES cells. The embryonic germ cell can be established by culturing primordial germ cells in the presence of substances such as LIF, bFGF, stem cell factor, etc. (Y. Matsui et al (1992), Cell, 70:.. 841-847; JL Resnick et al (1992), Nature, 359: 550 -551).

### (D) Induced pluripotent stem cells (iPS cells)

Induced pluripotent stem (iPS) cell is an artificial stem cell derived from a somatic cell, which can be produced by introducing a specific reprogramming factor in the form of a DNA or protein into a somatic cell, and show almost equivalent property (e.g., pluripotent differentiation and proliferation potency based on self-renewal) as ES cells (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. et al., Nat. Biotechnol. 26:101-106 (2008); WO2007/069666). The reprogramming factor may be constituted with a gene specifically expressed by ES cell, a gene product or non-coding RNA thereof, a gene playing an important role for the maintenance of undifferentiation of ES cell, a gene product or non-coding RNA thereof, or a low molecular weight compound. Examples of the gene contained in the reprogramming factor include Oct3/4, Sox2, Sox1, Sox3, Sox15, Soxl7, Klf4, Klf2, c-Myc, N-Myc, L-Myc, Nanog, Lin28, Fbxl5, ERas, ECAT15-2, Tell, beta-catenin, Lin28b, Sall1, Sall4, Esrrb, Nr5a2, Tbx3, Glis1 and the like. These reprogramming factors may be used alone or in combination. Examples of the combination of the reprogramming factors include combinations described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, WO2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26:2467-2474, Huangfu D, et al. (2008), Nat Biotechnol. 26:1269-1275, Shi Y, et al. (2008), Cell Stem Cell, 3, 568-574, Zhao Y, et al. (2008), Cell Stem Cell, 3:475-479, Marson A, (2008), Cell Stem Cell, 3, 132-135, Feng B, et al. (2009), Nat Cell Biol. 11:197-203, R. L. Judson et al., (2009), Nat. Biotech., 27:459-461, Lyssiotis C A, et al. (2009), Proc Natl Acad Sci USA. 106:8912-8917, Kim J B, et al. (2009), Nature. 461:649-643, Ichida J K, et al. (2009), Cell Stem Cell. 5:491-503, Heng J C, et al. (2010), Cell Stem Cell. 6:167-74, Han J, et al. (2010), Nature. 463:1096-100, Mali P, et al. (2010), Stem Cells. 28:713-720, and Maekawa M, et al. (2011), Nature. 474:225-9.

Examples of the above-mentioned reprogramming factor include, but are not limited to, factors used for enhancing the establishment efficiency, such as histone deacetylase (HDAC) inhibitors [e.g., low-molecular inhibitors such as valproic acid (VPA), trichostatin A, sodium butyrate, MC 1293, and M344, nucleic acid-based expression inhibitors such as siRNAs and shRNAs against HDAC (e.g., HDAC1 siRNA Smartpool® (Millipore), HuSH 29mer shRNA Constructs against HDAC1 (OriGene) and the like), and the like], MEK inhibitor (e.g., PD184352, PD98059, U0126, SL327 and PD0325901), Glycogen synthase kinase-3 inhibitor (e.g., Bio and CHIR99021), DNA methyl transferase inhibitors (e.g., 5-azacytidine), histone methyl transferase inhibitors [for example, low-molecular inhibitors such as BIX-01294, and nucleic acid-based expression inhibitors such as siRNAs and shRNAs against Suv39h1, Suv39h2, SetDB1 and G9a], L-channel calcium agonist (e.g., Bayk8644), butyric acid, TGFβ inhibitor or ALK5 inhibitor (e.g., LY364947, SB431542, 616453 and A-83-01), p53 inhibitor (e.g., siRNA and shRNA against p53), ARID3A inhibitor (e.g., siRNA and shRNA against ARID3A), miRNA such as miR-291-3p, miR-294, miR-295, mir-302 and the like, Wnt Signaling activating agent (e.g., soluble Wnt3a), neuropeptide Y, prostaglandins (e.g., prostaglandin E2 and prostaglandin J2), hTERT, SV40LT, UTF1, IRX6, GLIS1, PITX2, DMRTB1 and the like. In the present specification, these factors used for enhancing the establishment efficiency are not particularly 5 distinguished from the reprogramming factor.

When in the form of a protein, a reprogramming factor may be introduced into a somatic cell by a method, for example, lipofection, fusion with cell penetrating peptide (e.g., TAT derived from HIV and polyarginine), microinjection and the like.

When in the form of a DNA, a reprogramming factor may be introduced into a somatic cell by the method of, for example, vector of virus, plasmid, artificial chromosome and the like, lipofection, liposome, microinjection and the like. Examples of the virus vector include retrovirus vector, lentivirus vector (Cell, 126, pp. 663-676, 2006; Cell, 131, pp. 861-872, 2007; Science, 318, pp. 1917-1920, 2007), adenovirus vector (Science, 322, 945-949, 2008), adeno-associated virus vector, vector of Hemagglutinating Virus of Japan (WO 2010/008054) and the like. Examples of the artificial chromosome vector include human artificial chromosome (HAC), yeast artificial chromosome (YAC), bacterial artificial chromosome (BAC, PAC) and the like. As the plasmid, plasmids for mammalian cells can be used (Science, 322:949-953, 2008). The vector can contain regulatory sequences of promoter, enhancer, ribosome binding sequence, terminator, polyadenylation site and the like so that a nuclear reprogramming substance can be expressed and further, where necessary, a selection marker sequence of a drug resistance gene (e.g., kanamycin resistance gene, ampicillin resistance gene, puromycin resistance gene and the like), thymidine kinase gene, diphtheria toxin gene and the like, a reporter gene sequence of green fluorescent protein (GFP), β glucuronidase (GUS), FLAG and the like, and the like. Moreover, the above-mentioned vector may have a LoxP sequence before and after thereof to simultaneously cut out a gene encoding a reprogramming factor or a gene encoding a reprogramming factor bound to the promoter, after introduction into a somatic cell.

When in the form of RNA, for example, it may be introduced into a somatic cell by a means of lipofection, microinjection and the like, and RNA incorporating 5-methylcytidine and pseudouridine (TriLink Biotechnologies) may be used to suppress degradation (Warren L, (2010) Cell Stem Cell. 7:618-630).

Examples of the culture medium for inducing iPS cell include 10 to 15% FBS-containing DMEM, DMEM/F12 or DME culture medium (these culture media can further contain LIF, penicillin/streptomycin, puromycin, L-glutamine, nonessential amino acids, β-mercaptoethanol and the like as appropriate) or a commercially available culture medium [for example, culture medium for mouse ES cell culture (TX-WES culture medium, Thromb-X), culture medium for primate ES cell (culture medium for primate ES/iPS cell, Reprocell), serum-free medium (mTeSR, Stemcell Technologies)] and the like.

Examples of the culture method include contacting a somatic cell with a reprogramming factor on 10% FBS-containing DMEM or DMEM/F12 culture medium at 37°C in the presence of 5% CO₂ and culturing for about 4 to 7 days, thereafter reseeding the cells on feeder cells (e.g., mitomycin C-treated STO cells, SNL cells etc.), and culturing the cells in a bFGF-containing culture medium for primate ES cell from about 10 days after the contact of the somatic cell and the reprogramming factor, whereby ES-like colonies can be obtained after about 30 to about 45 days or longer from the contact.

Alternatively, the cells are cultured on feeder cells (e.g., mitomycin C-treated STO cells, SNL cells etc.) at 37° C. in the presence of 5% CO₂ in a 10% FBS-containing DMEM culture medium (which can further contain LIF, penicillin/streptomycin, puromycin, L-glutamine, nonessential amino acids, β-mercaptoethanol and the like as appropriate), whereby ES-like colonies can be obtained after about 25 to about 30 days or longer. Desirably, a method using a somatic cell itself to be reprogrammed, or an extracellular substrate (e.g., Laminin-5 (WO2009/123349) and Matrigel (BD)), instead of the feeder cells (Takahashi K, et al. (2009), PLoS One. 4:e8067 or WO2010/137746).

Besides the above, a culture method using a serum-free medium can also be recited as an example (Sun N, et al. (2009), Proc Natl Acad Sci USA. 106:15720-15725). Furthermore, to enhance establishment efficiency, an iPS cell may be established under hypoxic conditions (oxygen concentration of not less than 0.1% and not more than 15%) (Yoshida Y, et al. (2009), Cell Stem Cell. 5:237-241 or WO2010/013845), can be mentioned.

The culture medium is exchanged with a fresh culture medium once a day between the above-mentioned cultures, from day 2 from the start of the culture. While the cell number of the somatic cells used for nuclear reprogramming is not limited, it is about 5×10³ to about 5×10⁶ cells per 100 cm² culture dish.

The iPS cell can be selected based on the shape of the formed colony. When a drug resistance gene which is expressed in association with a gene (e.g., Oct3/4, Nanog) expressed when a somatic cell is reprogrammed is introduced as a marker gene, an established iPS cell can be selected by culturing in a culture medium (selection culture medium) containing a corresponding drug. When the marker gene is a fluorescent protein gene, iPS cell can be selected by observation with a fluorescence microscope, when it is a luminescent enzyme gene, iPS cell can be selected by adding a luminescent substrate, and when it is a chromogenic enzyme gene, iPS cell can be selected by adding a chromogenic substrate.

### (E) Non-human cloned embryo-derived ES cells obtain by nuclear transplantation (ntES cells)

The non-human nt ES cells are ES cells derived from a cloned embryo, which are prepared by a nuclear transplantation technique, and have almost the same properties as those of fertilized egg-derived ES cells (T. Wakayama et al. (2001), Science, 292:740-743; S. Wakayama et al. (2005), Biol. Reprod., 72:932-936; J. Byrne et al. (2007), Nature, 450:497-502). That is to say, non-human ES (nuclear transfer ES) cells are ES cells established from the inner cell mass of a blastocyst derived from a cloned embryo obtained by substituting the nucleus of an unfertilized egg with the nucleus of a somatic cell. For preparation of such non-human nt ES cells, a combination of a nuclear transplantation technique (J.B. Cibelli et al. (1998), Nature Biotechnol., 16:642-646) with the ES cell preparation technique (as described above) is (Wakayama Sayaka et al. (2008), Experimental Medicine, Vol. 26, No. 5 (special edition), 47-52 pages). In nuclear transplantation, the nucleus of a somatic cell is injected into the enucleated unfertilized egg of a mammal, and the egg is then cultured for several hours, so that it can be initialized.

### (F) Multilineage-differentiating Stress Enduring cells (Muse cells)

Muse cells are pluripotent stem cells prepared by the method described in WO2011/007900. In particular, a trypsinization of fibroblasts or bone marrow stromal cells for a long time, preferably for 8 hours or 16 hours, followed by suspension culture, can result in a production of SSEA-3 and CD105-positive cells having a pluripotency, i.e., Muse cells.

The term "somatic cells" used in the present specification means any animal cells excluding germ line cells and totipotent cells such as ovum, oocyte, ES cells and the like. The animal is preferably mammals including human and more preferably human and mouse. Somatic cells unlimitatively encompass any of somatic cells of fetuses, somatic cells of neonates, and mature healthy or pathogenic somatic cells. Specific examples of the somatic cells include (1) tissue stem cells (somatic stem cells) such as neural stem cell, hematopoietic stem cell, mesenchymal stem cell, dental pulp stem cell and the like, (2) tissue progenitor cells, (3) differentiated cells such as lymphocyte, epithelial cell, endothelial cell, myocyte, fibroblast (skin cells etc.), hair cell, hepatocyte, gastric mucosal cell, enterocyte, splenocyte, pancreatic cell (pancreatic exocrine cell etc.), brain cell, lung cell, renal cell and adipocyte and the like, and the like.

### <Method for screening for prophylactic or therapeutic agent for tauopathy>

The present invention provides a method for screening for a prophylactic or therapeutic agent for tauopathy (hereinafter, referred to as method II), comprising the following steps (1) to (4):
(1) contacting first neurons having a mutant MAPT gene with a test substance, followed by culture;
(2) contacting the culture supernatant of the first neurons obtained in the step (1) with second neurons;
(3) measuring any one index selected from the group consisting of the following (a) to (d) in the second neurons obtained in the step (2):
   (a) the amount of an intracellular tau oligomer,
   (b) the frequency of depolarization,
   (c) an intracellular calcium ion concentration, and
   (d) the number of living cells; and
(4) selecting the test substance as a prophylactic or therapeutic agent for tauopathy when the value of (a), (b), or (c) measured in the step (3) after the contact of the first neurons with the test substance in the step (1) is lower than the value obtained without the contact of the first neurons with the test substance in the step (1), and/or when the value of (d) measured in the step (3) after the contact of the first neurons with the test substance in the step (1) is higher than the value obtained without the contact of the first neurons with the test substance in the step (1).

The present invention further provides a method for screening for a prophylactic or therapeutic agent for tauopathy (hereinafter, referred to as method III), comprising the following steps (1) to (4):
(1) contacting a culture supernatant of first neurons having a mutant MAPT gene with second neurons;
(2) further contacting the second neurons with a test substance;
(3) measuring any one index selected from the group consisting of the following (a) to (d) in the second neurons obtained in the step (2):
   (a) the amount of an intracellular tau oligomer,
   (b) the frequency of depolarization,
   (c) an intracellular calcium ion concentration, and
   (d) the number of living cells; and
(4) selecting the test substance as a prophylactic or therapeutic agent for tauopathy when the value of (a), (b), or (c) measured in the step (3) after the contact of the second neurons with the test substance in the step (2) is lower than the value obtained without the contact of the second neurons with the test substance in the step (2), and/or when the value of (d) measured in the step (3) after the contact of the second neurons with the test substance in the step (2) is higher than the value obtained without the contact of the second neurons with the test substance in the step (2).

The method II is a method for screening for a test substance capable of suppressing (a) the secretion of a tau oligomer from the neurons having a mutant MAPT gene, and suppressing the ability of the secreted tau oligomer to propagate neurodegeneration.

The method III is a method for screening for an agent capable of suppressing neurodegeneration intracellularly caused in normal neurons exposed to a tau oligomer. The "second neurons" (i.e., normal neurons added to the culture supernatant of the neurons having a mutant MAPT gene) in the method III can be regarded as models of brain neurons of an initial stage to middle stage tauopathy patient with advanced neurodegeneration propagation.

### Target disease

The screening method according to the present invention is useful for searching for a compound that can serve as a prophylactic or therapeutic agent of tauopathy, or a lead compound or a seed compound thereof. Typical examples of the tauopathy include Alzheimer's disease (AD), frontotemporal dementia with parkinsonism linked to chromosome 17 (FTDP-17), frontotemporal dementia (FTD), Pick's disease, progressive supranuclear palsy (PSP), corticobasal degeneration (CBD), argyrophilic grain dementia (argyrophilic grain disease), dementia with neurofibrillary tangles, and diffuse neurofibrillary tangle with calcification (DNTC). Among these diseases, AD, FTDP-17, and FTD are particularly preferred as target diseases of the screening method according to the present invention because many patients having mutations in the MAPT gene have been reported.

### Test substance

Examples of the test substance to be subjected to the screening method of the present invention include proteins, peptides, antibodies, non-peptide compounds, synthetic compounds, synthetic low-molecular compounds, natural compounds, cell extracts, plant extracts, animal tissue extracts, plasma, marine organism-derived extracts, cell culture supernatants, and microbial fermentation products. These substances may be novel or may be known in the art.

The test substance may be obtained by using any of a number of approaches in combinatorial library methods known in the art, such as (1) the biological library method, (2) the synthetic library method using deconvolution, (3) the "one-bead one-compound" library method and (4) the synthetic library method using affinity chromatography selection. Application of the biological library method using affinity chromatography selection is limited to peptide libraries, but the other 4 types of approaches can be applied to low-molecular compound libraries of peptides, non-peptide oligomers or compounds (Lam, Anticancer Drug Des. 12:145-67, 1997). Examples of synthetic methods of molecular libraries can be found in the art (DeWitt et al. (1993) Proc. Natl. Acad. Sci. USA 90:; Erb et al. (1994) Proc. Natl. Acad. Sci. USA 91: 11422-6; Zuckermann et al. (1994) J. Med. Chem. 37: 2678-85; Cho et al. (1993) Science 261: 1303-5; Carell et al. (1994) Angew. Chem. Int. Ed. Engl. 33: 2059; Carell et al. (1994) Angew. Chem. Int. Ed. Engl. 33: 2061; Gallop et al. (1994) J. Med. Chem. 37: 1233-51). The compound libraries may be prepared as solutions (see Houghten, Bio/Techniques 13: 412-21, 1992) or beads (Lam, Nature 354:82-4, 1991), chips (Fodor, Nature 364: 555-6, 1993), bacteria (US 5,223,409 B), spores (US 5,571,698 B , US 5,403,484 B and US 5,223,409 B), plasmids (Cull et al. (1992) Proc. Natl. Acad. Sci. USA 89: 1865-9) or phages (Scott and Smith (1990) Science 249: 386-90; Devlin (1990) Science 249: 404-6; Cwirla et al. (1990) Proc. Natl. Acad. Sci. USA 87: 6378-82; Felici (1991) J. Mol. Biol. 222: 301-10; US 2002103360 B).

In the present invention, the contact of the test substance with the neurons may be performed by adding the test substance to a culture solution of the neurons. The contact is not particularly limited as long as change in the index can be confirmed in the time. Examples of the contact time include 1 day or longer, 2 days or longer, 3 days or longer, 4 days or longer, 5 days or longer, 6 days or longer, and 7 days or longer. The contact time is more preferably 2 days. The concentration of the test substance to be added can be appropriately regulated by the type (solubility, toxicity, etc.) of the compound.

In the present invention, the culture solution of the neurons for use in the contact of the test substance with the neurons is not particularly limited as long as the neurons can be cultured in the medium. Examples thereof include the media for neural differentiation mentioned above.

In the present invention, the temperature contacting neurons with a test substance is not particularly limited, and about 30 to 40°C, preferably about 37°C, in an air atmosphere containing CO₂. The CO₂ concentration is preferably about 2 to 5%.

### Culture supernatant of neuron

In the present invention, the culture supernatant of the neurons refers to a supernatant obtained by the low-speed centrifugation (e.g., centrifugation by centrifugal force on the order of 1,000 x g) of the medium in which the neurons have been cultured, for the purpose of removing cellular debris.

The culture supernatant of the neurons having a mutant MAPT gene according to the present invention has the activity of inducing various pathological changes associated with tauopathy against normal neurons and can be used as a neurodegeneration inducer.

The time when the culture supernatant is prepared from the neurons is not particularly limited. When the neurons are, for example, mouse neurons, medium replacement is performed for the neurons of days 3 to 10, more preferably days 4 to 9, most preferably days 5 to 8, and the neurons are then cultured for 1 day to 3 days, more preferably 2 days to 3 days, most preferably 2 days, in the medium, from which the culture supernatant may be prepared. When the neurons are human neurons, medium replacement is performed for the neurons of days 8 to 21, more preferably days 10 to 16, most preferably days 12 to 15, and the neurons are then cultured for 1 day to 3 days, more preferably 2 days to 3 days, most preferably 2 days, in the medium, from which the culture supernatant may be prepared. The day represents the number of days from the day at which the expression of Neurogenin 2 is induced in pluripotent stem cells having the mutant MAPT gene (= day 0).

In the present invention, the concentration of the culture supernatant to be contacted with the neurons is not particularly limited as long as the concentration allows neurodegeneration to be propagated to the neurons. It is preferred to add, for example, 40 to 80% (v/v), preferably 50 to 70% (v/v), most preferably approximately 50% (v/v) (final concentration) of the culture supernatant to the medium of the neurons.

In the present invention, the neurons used in the production of the culture supernatant and the neurons to be contacted with the culture supernatant do not have to be neurons of the same animal species. For example, a culture supernatant of mouse neurons may be contacted with human neurons, or a culture supernatant of human neurons may be contacted with mouse neurons.

The culture supernatant provided by the present invention may be cryopreserved for use.

### Tau oligomer

In the present invention, the "tau oligomer" means an associate in which 3 to 50 tau monomers are associated. The tau oligomer may be a soluble tau oligomer or may be an insoluble tau oligomer. The soluble tau oligomer may be an oligomer recovered into a supernatant by centrifugation operation at approximately 10,000 x g after dissolution in 1% polyoxyethylene(10) octylphenyl ether (Triton X-100, CAS No. 9002-93-1). The insoluble tau oligomer may be an oligomer recovered as precipitates after the centrifugation operation. The tau oligomer excludes tau fibrils (e.g., PHF), which are filaments in which insoluble tau oligomers are bound to each other.

In the present invention, the tau oligomer may undergo various chemical modifications including phosphorylation. Also, the tau oligomer may form a complex with another protein and/or nucleic acid (DNA or RNA).

### Tau oligomer isolated from culture supernatant of neuron

In the present invention, a tau oligomer isolated from the culture supernatant of the neurons having a mutant MAPT gene can be used as a neurodegenerative agent, as with the culture supernatant.

The isolation of the tau oligomer from the culture supernatant can be performed by use of a method well known to those skilled in the art. The tau oligomer may be isolated by use of, for example, an immunoprecipitation method using an anti-tau oligomer antibody mentioned later, or a biochemical fractionation method (see e.g., Non-Patent Document 5).

In the present invention, the concentration of the tau oligomer to be contacted with the neurons is not particularly limited as long as the concentration allows neurodegeneration to be propagated to the neurons. It is preferred to add, for example, 0.05 to 10.0 ng/ml, preferably 0.1 to 5.0 ng/ml, more preferably 0.2 to 2.5 ng/ml, most preferably 0.5 to 1.0 ng/ml (final concentration) of the tau oligomer to the medium of the neurons.

The amount of the tau oligomer contained in the culture supernatant of tauopathy iNs differs largely depending on a culture period, a cell density, and the number of days from differentiation, etc. As a guideline, the amount of the tau oligomer is approximately 5 to 10 ng/ml in a 3-day culture supernatant of mouse tauopathy iNs and approximately 1 to 5 ng/ml in a 5-day culture supernatant of human tauopathy iNs. These values are results of quantification based on a calibration curve of recombinant tau monomers by use of ELISA.

### Artificial tau oligomer

An artificial tau oligomer may be used as an inducer of tau-mediated neurodegeneration. The artificial tau oligomer is a tau oligomer obtained by the *in vitro* artificial oligomerization of tau monomers. The tau monomers may be recombinant tau produced by use of a DNA recombination technique or may be tau isolated and purified from cells or tissues. These tau monomers can be oligomerized *in vitro* by use of a method well known to those skilled in the art. Examples thereof include a method which involves incubating the tau monomers in the presence of heparin (Sahara N. and Takashima A, EMBO J., 26: 5143-52, 2007), and a method which involves oligomerizing the tau monomers with an Aβ oligomer as a core (Cristian A., et al., FASEB J., 26: 1946-59, 2012).

The concentration of the artificial tau oligomer to be contacted with the neurons is not particularly limited as long as the concentration allows neurodegeneration to be propagated to the neurons. In the case of producing the artificial tau oligomer from, for example, recombinant tau monomers, it is preferred to add 0.05 to 10.0 µg/ml, preferably 0.1 to 5.0 µg/ml, more preferably 0.2 to 2.5 µg/ml, most preferably 0.5 to 1.0 µg/ml (final concentration) of the artificial tau oligomer to the medium of the neurons.

### Index

Each index that can be used in the screening method of the present invention will be described in detail.

### <Amount of tau oligomer in culture supernatant>

The amount of a tau oligomer in a medium (more preferably a culture supernatant) of the neurons having a mutant MAPT gene can be used as an index for screening for a prophylactic or therapeutic agent for tauopathy. This is because the tau oligomer secreted by the neurons having a mutant MAPT gene has the activity of inducing the cell death of their surrounding neurons, and their own (= neurons having a mutant MAPT gene) cell death also correlates with the amount of the tau oligomer in the medium.

The method for measuring the amount of a tau oligomer in a culture supernatant is not particularly limited, and a method routinely used by those skilled in the art can be used. The amount of a tau oligomer in a culture supernatant may be measured by use of, for example, ELISA (enzyme-linked immunosorbent assay). Examples of such a method include sandwich ELISA using a tau oligometric complex 1 antibody (epitope: 209-224th human tau amino acids; hereinafter, referred to as a TOC1 antibody; Non-Patent Documents 12 and 13) as an immobilized antibody, and Tau 13 (epitope: 20-35th human tau amino acids, Abcam plc), Tau 12 (epitope: 9-13th human tau amino acids, Merck Millipore), human tau (Dako/Agilent Technologies, Inc.), or the like as a detection antibody. Alternatively, the amount of a tau oligomer in a culture supernatant may be measured by quantifying signals obtained by dot blot using an anti-tau oligomer antibody (e.g., a TOC1 antibody or an antibody described in WO2011/026031). Furthermore, this measurement may be performed by quantifying signals of approximately trimeric to 50-meric molecular species by Western blot using a tau antibody routinely used. The TOC1 antibody may be prepared by those skilled in the art according to a method described in, for example, Non-Patent Document 12.

The neurons having a mutant MAPT gene can be contacted with the test substance, followed by culture, and measuring the amount of a tau oligomer in a culture supernatant as the amount of a tau oligomer secreted into the medium (culture supernatant) during the culture period after the contact with the test substance.

The phrase "the value of the amount of a tau oligomer in a culture supernatant is lower" refers to a value of 80%, 70%, 60%, 50%, or 40% or lower with respect to the amount of a tau oligomer in a culture supernatant of the neurons uncontacted with the test substance (control value).

### <Amount of intracellular tau oligomer>

In the present invention, the amount of an intracellular tau oligomer in the second neurons contacted with the culture supernatant of the first neurons having a mutant MAPT gene (the method II or III), or the second neurons contacted with the tau oligomer isolated from the culture supernatant (method III) can be used as an index for screening for a prophylactic or therapeutic agent for tauopathy. The "amount of an intracellular tau oligomer" is preferably the amount of the tau oligomer bound to lipid raft on the cell membranes of the cells. This is because the tau oligomer secreted from the neurons having a mutant MAPT gene has been found by the present inventors to specifically bind to lipid raft on these neurons themselves or their surrounding neurons.

The lipid raft is a domain on a cell membrane rich in sterol and sphingolipid (Simons K., et al., Nature, 387: 569-72, 1997). The amount of the tau oligomer bound to the lipid raft may be the amount of the tau oligomer extracellularly bound to the lipid raft, the amount of the tau oligomer intracellularly bound to the lipid raft, or the total sum thereof. The amount of the tau oligomer extracellularly bound to the lipid raft is particularly preferred.

The amount of an intracellular tau oligomer can be measured by lysing the cells by a method well known to those skilled in the art and measuring the amount of the tau oligomer in the lysates using the anti-tau oligomer antibody mentioned above. The measurement method can be performed according to a protein assay method known in the art. Examples thereof include immunological assay methods such as Western blotting, dot blot, immunoprecipitation, EIA (enzyme immunoassay), ELISA (enzyme-linked immunosorbent assay), RIA (radioimmunoassay), FIA (fluorescent immunoassay), and immunocytochemistry.

The amount of the tau oligomer bound to lipid raft can be determined, for example, by biochemically isolating the lipid raft from the cells by use of a method well known to those skilled in the art (Gil C., et al., Biochem. Biophys. Commun., 348: 1334-42, 2006; Song K., et al., J. Biol. Chem., 271: 9690-97, 1996; and Iwabuchi K., et al., J. Biol. Chem., 273: 33766-73, 1998), followed by the aforementioned method for measuring the amount of a tau oligomer. Particularly, examples of the method for determining the amount of the tau oligomer extracellularly bound to the lipid raft include a method which involves subjecting the cells to treatment with CTB (cholera toxin B, cholera toxin beta subunit) covalently bound with a reporter protein and immunostaining using an anti-tau oligomer antibody, followed by quantification as the number of sites where signals obtained from the reporter protein and signals of the immunostaining are colocalized. Since CTB specifically binds to ganglioside GM1, which is a marker molecule of lipid raft, CTB covalently bound with various reporter proteins (e.g., fluorescent dyes such as GFP and FITC, enzymes such as HRP, and tags such as FLAG), and detection kits (e.g., Vybrant Lipid Raft Labeling Kit, Molecular Probes, Inc.) can be utilized as approaches for visualizing the lipid raft. The number of the colocalized signals can be visually measured and may be automatically measured using a cell image analysis apparatus (e.g., IN Cell Analyzer, GE Healthcare Japan Corp.).

In the present invention, the phrase "the value of the amount of an intracellular tau oligomer is lower" refers to a value of 80%, 70%, 60%, 50%, or 40% or lower with respect to the amount of an intracellular tau oligomer in the neurons uncontacted with the test substance (control value).

### <Frequency of depolarization and intracellular calcium ion concentration>

In the present invention, the frequency of depolarization and/or an intracellular calcium concentration in the the second neurons contacted with the culture supernatant of the first neurons having a mutant MAPT gene (the method II or III), or the second neurons contacted with the tau oligomer isolated from the culture supernatant (method III)
can be used as an index for screening for a prophylactic or therapeutic agent for tauopathy. This is because the neurons having a mutant MAPT gene exhibit increased excitability, and the suppression of the excitability suppresses both of the cell death of the cells and the propagation of neurodegeneration by the culture supernatant.

In the present invention, the frequency of depolarization can be measured, for example, as the rate of firing that occurs under conditions involving no artificial stimulation from the outside (spontaneous firing), or the rate of firing that occurs by artificial stimulation from the outside and/or the magnitude of change in membrane potential (action potential). The firing rate and/or the magnitude of change in membrane potential can be measured by use of a method routinely used by those skilled in the art and may be measured using, for example, a multielectrode array system (also referred to as a microelectrode array system; hereinafter, abbreviated to MEA) or a patch clamp method (preferably a whole-cell recording patch clamp method).

In the present invention, the intracellular calcium concentration can be preferably measured, for example, as the intracellular calcium concentration of the neurons after stimulation. The calcium concentration can be measured by use of a method routinely used by those skilled in the art and may be measured conveniently and highly sensitively by, for example, an intracellular calcium imaging method.

In the present invention, the phrase "the value of the frequency of depolarization or the intracellular calcium ion concentration is lower" refers to a value of 80%, 70%, 60%, 50%, or 40% or lower with respect to the frequency of depolarization or the intracellular calcium ion concentration of the neurons uncontacted with the test substance (control value).

### <The number of living cells>

In the present invention, the number of living cells (also referred to as the number of live neurons) of the second neurons contacted with the culture supernatant of the first neurons having a mutant MAPT gene (the method II or III), or the second neurons contacted with the tau oligomer isolated from the culture supernatant (method III) can be used as an index for screening for a prophylactic or therapeutic agent for tauopathy.

A measurement of the number of living cells is not limited and may be carried out by a method well known to those skilled in the art. For example, cells are immunestained with an antibody to a marker protein for neurons such as β-III tubulin, NeuN, N-CAM, and MAP2, followed by counting the number of cells positive for the protein. The counting may be carried out visually or using an automated cell counting device (e.g., IN Cell Analyzer). Alternatively, the number of cells may be calculated as the reciprocal of the number of dead cells. Measurement of the number of dead cells can be carried out, for example, by a method of measuring the activity of LDH (Lactate Dehydrogenase) in the medium, MTT (3-(4,5-di-methylthiazol-2-yl)-2,5-diphenyltetrazolium bromide, yellow tetrazole) method, WST (Water soluble Tetrazolium salts)-1 method, a method of measuring the absorbance using WST-8 method or, a method of counting cell number using a flow cytometer after staining the cells with TO (thiazole orange), PI (propidium iodide), 7AAD, calcein AM, or ethidium homodimer 1 (EthD-1). The number of positive cells may be counted using a flow cytometry.

In the present invention, the number of living cell is determined as "high", when the value of the cell is 1.4 times or more, 1.6 times or more, 1.8 times or more, 2 times or more, or 2.5 times or more than the value of the control cells which have not been contacted with the test substance.

### EXAMPLES

Hereinafter, the present invention will be described more specifically with reference to Examples. However, the present invention is not intended to be limited by these examples by any means.

In Examples below, a PS19 transgenic mouse harboring a P301S mutant human MAPT gene controlled by mouse prion promoter (Yoshiyama, et al., Neuron, 53: 337-351, 2007; available from The Jackson Laboratory under strain name: B6;C3-Tg(Prnp-MAPT*P301S)PS19Vle/J; see http://jaxmice.jax.org/strain/008169.html) is referred to as a "tauopathy mouse". This mouse is a tauopathy mouse model that manifests neurological dysfunction at the age of approximately 3 months and propagates NFT formation and neurodegeneration from the hippocampus to the cerebral neocortex over the age of approximately 6 to 12 months.

Hereinafter, iPSCs prepared from the tauopathy mouse and its non-transgenic litter are referred to as "tauopathy mouse-derived iPSCs" and "normal control mouse-derived iPSCs", respectively. Cells obtained by transferring a foreign neurogenin 2 gene to the chromosomes of these iPSCs are referred to as "tauopathy mouse-derived iPSCs for neural differentiation" and "normal control mouse-derived iPSCs for neural differentiation", respectively. Neurons obtained by inducing the expression of the foreign neurogenin 2 gene from these iPSCs for neural differentiation are referred to as "mouse tauopathy iNs" and "mouse normal iNs", respectively. The term "iNs" is an abbreviation of induced neurons. As shown in Examples, several days are required for mouse- and human-derived iPSCs for neural differentiation to differentiate into morphologically and functionally mature neurons after induction of foreign neurogenin 2 gene expression. In the specification of the present application, the iPSCs for neural differentiation after induction of foreign neurogenin 2 gene expression are referred to as iNs.

In Examples below, a familial FTDP-17 patient having a mutant MAPT gene having the substitution of cytosine at position 14 of intron 10 by thymine (intron 10 +14C > T) is referred to as a "FTDP-17 patient" or a "tauopathy patient". An iPSC line established from somatic cells of the patient is referred to as "tauopathy patient-derived iPSCs". A 201B7 line (see Takahashi K, et al., Cell, 131: 861-72, 2007) routinely used as human normal subject-derived iPSCs is referred to as "human normal subject-derived iPSCs". Cells obtained by transferring a foreign neurogenin 2 gene to the chromosomes of these iPSCs are referred to as "tauopathy patient-derived iPSCs for neural differentiation" and "human normal control-derived iPSCs for neural differentiation", respectively. Neurons obtained by inducing the expression of the foreign neurogenin 2 gene from these iPSCs for neural differentiation are referred to as "human tauopathy iNs" and "human normal iNs", respectively.

The term "day" in Examples of the present application represents the number of days from the day at which the expression of Neurogenin 2 is induced in iPSCs for neural differentiation (= day 0).

First, basic experimental approaches used in Examples of the present application will be described in detail.

### <Experimental approach>

### [Approach 1] Preparation of iPSC for neural differentiation from iPSC

A PB-TAC-ERN vector (see Non-Patent Document 11) was used in the preparation of a foreign neurogenin 2 gene to be transferred to iPSCs. The vector has an expression cassette that polycistronically expresses the gene of interest (in the present invention, the neurogenin 2 gene) and a reporter gene (mCherry) under the control of tetracycline-responsive promoter (tetracycline-inducible cassette) and an expression cassette that polycistronically expresses rtTA and neomycin resistance gene under the control of EF1α promoter, and further has PiggyBac transposon inverted repeat sequences upstream and downstream of the two expression cassettes. Accordingly, the cotransfection of cells with this vector and a vector that permits expression of PiggyBac transposase can yield cells having an insert of the two expression cassettes as a series of DNAs in the chromosomes of these cells (Non-Patent Document 11).

A polynucleotide encoding mouse Neurogenin 2 (NP_033848) or human Neurogenin 2 (SEQ ID NO: 4) was inserted to the tetracycline-inducible expression cassette of the PB-TAC-ERN vector to prepare a tetracycline-inducible mouse neurogenin 2 gene and human neurogenin 2 gene. Mouse iPSCs and human iPSCs were cotransfected with the mouse neurogenin 2 gene and the human neurogenin 2 gene, respectively, and a pCyl43 expression vector encoding PiggyBac transposase using lipofectamin LTX (Invitrogen Corp.). Then, the iPSCs were cultured in a neomycin (G418)-containing medium to select cells having an insert of the two expression cassettes in their chromosomes (i.e., iPSCs for neural differentiation). These cells were established as lines. In Examples below, the established lines of iPSCs for neural differentiation were used.

### [Approach 2] Preparation of iN from iPSC for neural differentiation

Mouse-derived iPSCs for neural differentiation were dissociated into single cells by treatment with 0.25% trypsin and then cultured for 1 hour on a gelatin-coated dish, and the supernatant (containing the mouse-derived iPSCs for neural differentiation) was inoculated onto a Matrigel-coated plastic plate or glass cover to remove feeder cells. On the next day (= day 0), the medium was replaced with a medium for neural differentiation (mixed medium of a DMEM/F12 medium and a neurobasal medium (mixing volume ratio: 1:1, both from Life Technologies Corp.) supplemented with 1% N2 supplement (Invitrogen Corp.), 2% B27 supplement (Invitrogen Corp.), 10 ng/ml BDNF (R&D Systems, Inc.), 10 ng/ml GDNF (R&D Systems, Inc.), and 10 ng/ml NT3 (R&D Systems, Inc.) supplemented with 1 µg/ml DOX (Clontech Laboratories, Inc.) to induce the expression of the foreign neurogenin 2 gene.

Human-derived iPSCs for neural differentiation were dissociated into single cells by treatment with Accutase (Innovative Cell Technologies, Inc.) and then inoculated onto a Matrigel-coated plastic plate or glass cover. On the next day (= day 0), the medium was replaced with the medium for neural differentiation (the same as that used for the mouse-derived iPSCs for neural differentiation) supplemented with 1 µg/ml DOX (Clontech Laboratories, Inc.) to induce the expression of the foreign neurogenin 2 gene.

### [Approach 3] Measurement of amount of tau oligomer by use of dot blot

Cells were recovered from the culture system of iNs, suspended in TBS (Tris buffered saline) containing a protease inhibitor and a phosphatase inhibitor, and then ultrasonicated to homogenize the cells. The cell homogenates were centrifuged (13,000 x g, 15 min), and the supernatant was recovered as cell extracts. The cell extracts from the iNs of each type were blotted at 1.2 µg protein/spot onto a nitrocellulose membrane (pore size: 0.45 µm, GE Healthcare Japan Corp.).

Also, a medium was recovered from the cell culture system and subjected to low-speed centrifugation (1,000 rpm, 3 min) to precipitate cellular debris. The obtained supernatant was recovered as a culture supernatant. A 500 µl aliquot of each culture supernatant was transferred to a tube with an ultrafiltration filter (Vivaspin, molecular weight cutoff: 10 kD, GE Healthcare Japan Corp.) and concentrated to 50 µl. 2 µl of each concentrated sample was blotted onto the nitrocellulose membrane.

The nitrocellulose membrane obtained by the method was treated with various tau antibodies according to a standard method, followed by detection (Western Lightning Plus-ECL, PerkinElmer, Inc.) and signal quantification (ImageQuant LAS4000, GE Healthcare Japan Corp.). Tau 12, Tau 5, and TOC1 antibodies were used a human tau-specific antibody, an antibody recognizing human tau and mouse tau, and a tau oligomer-specific antibody, respectively. In Examples of the present application, the TOC1 antibody (Non-Patent Document 13) used was provided by Dr. Nicholas Kanaan from Department of Translational Science and Molecular Medicine, College of Human Medicine, Michigan State University (USA).

### [Approach 4] Analysis of propagation of tau-mediated neurodegeneration Analysis using coculture system

Normal control mouse-derived iPSCs for neural differentiation were inoculated at 2 × 10⁵ cells/well to a 24-well plate. Meanwhile, normal control mouse-derived iPSCs for neural differentiation or tauopathy mouse-derived iPSCs for neural differentiation were inoculated at 1 × 10⁵ cells/well to wells for coculture (Cell Culture Insert, BD Bioscience) having a bottom made of a membrane having a pore size of 1 µm. The wells for coculture were placed in the medium in the 24-well plate inoculated with the normal control mouse-derived iPSCs for neural differentiation. In this state, the cells on the 24-well plate and the cells on the wells for coculture are in no direct contact therebetween and merely share the medium. After the placement, these cells were induced to differentiate according to the [Approach 2]. Seven days later, the wells for coculture were removed, and the cells on the 24-well plate were immunostained (βIII-tubulin and NeuN). The number of live neurons was measured using IN Cell Analyzer (GE Healthcare Japan Corp.).

### Analysis using culture supernatant

iPSCs for neural differentiation were inoculated at 2 × 10⁶ cells/well to a 6-well plate and induced to differentiate according to the [Approach 2]. At day 5 for mouse-derived iPSCs for neural differentiation and at day 12 or 14 for human-derived iPSCs for neural differentiation, the whole amount of the medium was replaced with fresh one. Two days later (48 hours later), the medium was recovered to prepare a culture supernatant. The obtained supernatant was mixed with an equal amount of the medium for neural differentiation, and half the amount thereof was replaced with the medium of the mouse normal iNs of day 8 or the human normal iNs of day 16. Two days later, these normal iNs were subjected to the measurement of the number of live neurons by immunostaining and the analysis of the amount of a tau oligomer by dot blot.

A culture supernatant immunodepleted of a tau oligomer was prepared by the following method: Dynabeads (VERITAS Corp.) bound with a TOC1 antibody were added to a culture supernatant recovered and prepared from the mouse tauopathy iNs of day 5, followed by incubation overnight at 4°C. Then, the beads were removed to obtain a culture supernatant immunodepleted of a tau oligomer. The culture supernatant immunodepleted of a tau oligomer was added to normal iNs in the same way as in a culture supernatant that was not immunodepleted of the tau oligomer.

### [Approach 5] Measurement of amount of tau oligomer bound to lipid raft

A tau oligomer extracellularly bound to lipid raft on neurons was assayed using Vybrant Lipid Raft Labeling Kit (Molecular Probes, Inc.). Cholera toxin subunit B (CTB) bound with Alexa 488 was added to a medium of iNs, followed by incubation (4°C, 15 min). Then, the cells were washed with PBS, and an anti-CTB antibody was added thereto and cross-linked to the cells (4°C, 15 min). The cells were washed with PBS, fixed in 4% paraformaldehyde (room temperature, 30 min), and then washed again with PBS. After subsequent blocking (30 min) using Block Ace (MEGMILK SNOW BRAND Co., Ltd.), the cells were treated with a TOC1 antibody (1:1,000, 4°C, overnight), washed with PBS, and then treated with a secondary antibody (room temperature, 1 hr). Signals were detected and analyzed using Delta Vision (Applied Precision Ltd.).

### [Approach 6] Measurement of spontaneous firing rate using MEA system

iPSCs for neural differentiation were inoculated to a culture dish for the exclusive use of MEA coated with poly-L-lysine and Matrigel, and induced to differentiate into neurons according to the [Approach 2]. At day 8 (mouse iNs) or day 12 (human iNs), extracellular electrical recordings were measured using a multielectrode array system (MEA60, Multi Channel Systems MCS GmbH). Data was obtained using MC_Rack software (extracellular electrical recordings). Offline Sorter ver. 3 (Plexon Inc.) was used in spike analysis. The spikes were sorted and then analyzed using Neuroexplorer software (Nex Technologies, LLC).

### [Approach 7] Measurement of intracellular calcium concentration by calcium imaging method

iPSCs for neural differentiation were dissociated into single cells, then inoculated at 5 × 10⁴ cells/well to a Matrigel-coated 96-well plate, and induced to differentiate into neurons according to the [Approach 2]. At day 7 for mouse-derived iPSCs for neural differentiation and at day 12 for human-derived iPSCs for neural differentiation, 5 µM Fluo-8AM (AAT Bioquest, Inc.) and 0.01% Pluronic F-127 (Sigma-Aldrich Co. LLC) were added to the medium, followed by incubation at 37°C for 30 minutes. Then, the cells were rinsed with PBS, and a phenol red-free neurobasal medium (Gibco/Thermo Fisher Scientific Inc.) was added thereto. The cells in each well were given electric stimulation of 50 Hz for 1 msec a total of 50 times. Change in the fluorescence intensity of the Fluo-8AM was measured (excitation: 340 nm/emission: 510 nm) using FDSS/µCELL (Functional Drug Screening System, Hamamatsu Photonics K.K.).

### [Example 1] Preparation of tauopathy mouse-derived iPSC and preparation of iN from the cell

MEFs (mouse embryonic fibroblasts) were collected from a tauopathy mouse or its non-transgenic litter, transfected with Oct3/4, Sox2, c-Myc, and Klf4 using a retrovirus vector, and then cultured using a medium for mouse ES cells (knockout DMEM medium (Life Technologies Corp.) supplemented with 15% FBS, LIF, β-mercaptoethanol, L-glutamine, nonessential amino acids, and penicillin/streptomycin) on feeder cells (SNL cells) to establish an iPSC line. The iPSCs prepared from any of the mice maintained the expression of an undifferentiation marker (Figures 1A and 1B), and the expression of the human mutant tau protein was confirmed in the tauopathy mouse-derived iPSCs (Figure 1C).

The two types of iPSCs were each transfected with the tetracycline-inducible neurogenin 2 gene to prepare iPSCs for neural differentiation ([Approach 1]). When the expression of the Neurogenin 2 was induced in the iPSCs for neural differentiation ([Approach 2]), cells expressing βIII-tubulin and NeuN appeared 12 hours later; cells extending neurites appeared 36 hours later; and 90% or more of the cells exhibited the morphology of mature neurons (morphology having βIII-tubulin/NeuN double positivity, thickening of a cell body, and neurites extended to 5 times or more the diameter of the cell body) 3 days later (Figure 1D, upper photograph). The expression of a neuron marker (N-CAM) on cell surface started earlier, and N-CAM-positive cells were observed 12 hours after DOX addition. By contrast, the expression of an undifferentiation marker (SSEA1) was immediately decreased after DOX addition, and all of the cells became SSEA1-negative 48 hours later (Figure 1D, lower photograph). Furthermore, many vGLT1 (vesicular glutamate transporter 1)-positive granules were found in the neurites of these iNs (Figure 1E). At day 7, typical action potential was measured, confirming that the cells were functionally matured as neurons (Figure 1F).

The two types of iNs did not generate action potential in the presence of a NMDA glutamate receptor antagonist AP-5 (D-(-)-2-amino-5-phosphononpentanoic acid) and a non-NMDA glutamate receptor antagonist CNOX (6-cyano-7-nitroquinoxaline-2,3-dione) (Figure 1G). Accordingly, these iNs were confirmed to be glutamatergic neurons. Between the two types of iNs, no significant difference was found in the number of morphologically mature neurons at day 3 and the number of neurons that generated action potential at day 7.

These results demonstrated that when iPSCs prepared from mouse cells having a mutant MAPT gene are transfected with a neurogenin 2 gene and induced to express the gene, the iPSCs become morphologically mature neurons in approximately 3 days and are also functionally matured as glutamatergic neurons in approximately 7 days, as with iPSCs prepared from normal mouse cells.

### [Example 2] Pathological condition of tauopathy manifested by mouse tauopathy iN

It has been reported that neurons differentiated by induction from pluripotent stem cells by the expression of a foreign neurogenin 2 gene express a pyramidal cell marker CaMKII (Ca²⁺/calmodulin-dependent protein kinase II) and a neuron marker Satb2 (special AT-rich sequence-binding protein 2) of the layers II and III of the cerebral cortex, and are thus pyramidal cells of the cerebral cortex (Patent Document 7 and Non-Patent Document 10). As a result of immunostaining, mouse tauopathy iNs express CaMKII and Satb2, as with mouse normal iNs (Figure 2A), demonstrating that the cells were differentiated into pyramidal cells of the cerebral cortex. The pyramidal cells are neurons damaged mainly in AD or FTDP-17.

Next, tau hyperphosphorylation, the presence or absence of a tau oligomer, the number of synapses, and cell death were analyzed. Hyperphosphorylated tau, which is hardly detected in mouse normal iNs, was detected in cell extracts of tauopathy iNs of day 8 (Figure 2E). In addition, tau oligomers accumulated in the cell bodies and neurites of tauopathy iNs of day 10, in such a large amount that the tau oligomers were detectable by immunostaining (Figure 2B, right panel). By contrast, no tau oligomer was detected in mouse normal iNs (Figure 2B, left panel).

Subsequently, the iNs were double-immunostained with antibodies against marker proteins of presynaptic cells (Synapsin I) and postsynaptic cells (Drebrin) to visualize synapse sites (= sites double-stained with Synapsin and Drebrin) (Figure 2C). As a result of measuring the number of the synapse sites (= the number of synapses), the number of synapses per mouse tauopathy iN was significantly lower than the number of synapses per mouse normal iN (Figure 2D). As a result of further measuring the number of living cells at days 7 and 10, the mouse normal iNs had no significant difference in the number of living cells between day 7 and day 10, whereas the number of living cells at day 10 of the mouse tauopathy iNs was decreased to approximately 30 to 40% of the number of living cells at day 7 (Figure 2F). The number of living cells of the mouse tauopathy iNs tended to be abruptly decreased at day 9 or later. Furthermore, the mouse normal iNs had no significant difference in lactate dehydrogenase (LDH) level in a medium between day 7 and day 10, whereas the LDH level at day 10 of the mouse tauopathy iNs was approximately 160% of the LDH level at day 7 (Figure 2F).

Accordingly, it was shown that in the mouse tauopathy iNs according to the present invention, tau hyperphosphorylation and oligomerization occur spontaneously, and pathological changes, such as the accumulation of tau oligomers in cell bodies and neurites, and decrease in the number of synapses, characteristic of tauopathy progress automatically, leading to cell death.

The present inventors further found that a tau oligomer is extracellularly secreted by mouse tauopathy iNs.

A culture supernatant and cell extracts were prepared from the culture system of iNs of day 8, and the amount of a tau oligomer, the amount of human tau, and the total amount of tau contained in the culture supernatant or the cell extracts were analyzed by dot blot. For the culture system of the normal mouse iNs, no tau oligomer was substantially detected in the cell extracts and the culture supernatant (Figure 2H, signals found in the normal iNs were nonspecific signals from antibodies). By contrast, for the culture system of the mouse tauopathy iNs, the tau oligomer was contained not only in the cell extracts but in the culture supernatant of the medium (Figure 2H). Although it has previously known that monomeric tau is secreted from neurons, there has been no report on the secretion of oligomerized tau. Thus, this phenomenon was found for the first time by the present inventors.

Thus, next, the functions of the tau oligomer secreted by the mouse tauopathy iNs were analyzed.

### [Example 3] Induction of neuronal death by extracellular tau oligomer

Mouse tauopathy iNs and mouse normal iNs were cocultured in no direct contact between the cells, and change in the normal iNs was analyzed. Mouse tauopathy iNs were cultured on wells for coculture having a bottom made of a membrane having a pore size of 1 µm, and the wells were placed in the medium of a mouse normal iN culture system (Figure 3A, right diagram). As a control, wells for coculture containing cultured mouse normal iNs were placed in the medium of a mouse normal iN culture system (Figure 3A, left diagram).

These cells were cocultured for 7 days (day 0-day 7). Then, the normal iNs were double-immunostained with β-III tubulin and NeuN (Figure 3B), and the number of living cells was measured (Figure 3C). As shown in Figure 3C, the number of living cells of the normal iNs cocultured with the mouse tauopathy iNs was approximately 80% of the number of living cells of the control cocultured with the mouse normal iNs. Accordingly, approximately 20% of cells among the normal iNs cocultured with the mouse tauopathy iNs were found to die.

Next, only a culture supernatant of mouse tauopathy iNs was added to the culture system of mouse normal iNs, and change in the normal iNs was analyzed. As shown in Figure 3D, a culture supernatant of mouse tauopathy iNs or normal iNs of days 5 to 7 was added (final concentration: 50% (v/v)) to a medium of mouse normal iNs of day 8 and then cultured for 2 days, and the number of living cells at day 10 was measured. As a result, only approximately 50 to 60% of the number of living cells was able to be confirmed in the normal iNs supplemented with the culture supernatant of the mouse tauopathy iNs as compared with the control supplemented with the culture supernatant of the mouse normal iNs (Figure 3E, left panel, and Figure 3F, left graph). By contrast, in the case of adding the mouse tauopathy iN culture supernatant immunodepleted of a tau oligomer, 80% or more of the cells survived, as compared with the control (Figure 3E, right panel, and Figure 3F, right graph).

These results demonstrated that a tau oligomer extracellularly secreted by the mouse tauopathy iNs according to the present invention has the activity of inducing the cell death of normal neurons.

### [Example 4] Binding of tau oligomer to lipid raft and propagation of neurodegeneration

The mechanism under which a tau oligomer secreted into a medium induces the cell death of normal neurons was analyzed.

First, the behavior of an extracellularly secreted tau oligomer was analyzed in the culture system of mouse tauopathy iNs. Figure 4A shows results of double-(immuno)staining normal or tauopathy iNs using CTB (cholera toxin B) specifically binding to lipid raft and an anti-tau oligomer antibody. Many tau oligomers colocalized with signals of CTB were confirmed in the mouse tauopathy iNs (Figure 4A, right panel), whereas no signal from a tau oligomer was detected in the culture system of the mouse normal iNs (Figure 4A, left panel).

Next, a culture supernatant of mouse tauopathy iNs was added to a medium of mouse normal iNs, followed by CTB/anti-tau oligomer antibody double staining two days later. As a result, many tau oligomers colocalized with lipid raft on the normal iNs were detected (Figure 4B, right panel). Cell extracts were prepared from the normal iNs supplemented with the culture supernatant of the mouse tauopathy iNs, and analyzed by dot blot. As a result, signals of the human tau protein were increased by approximately 1.4 times (Figure 4C, middle panel, and Figure 4E), and tau oligomer signals were increased by approximately 2.7 times (Figure 4C, upper panel, Figure 4E), as compared with normal iNs supplemented with a culture supernatant of mouse normal iNs.

Accordingly, the tau oligomer secreted by the mouse tauopathy iNs was found to bind to lipid raft on the cell membranes of mouse normal iNs. The possibility was further suggested that a new tau oligomer is formed in the tau oligomer-bound normal iNs. This suggested that the cell death of mouse normal iNs induced by a tau oligomer secreted by mouse tauopathy iNs is cell death involving tau oligomerization, as with the cell death of mouse tauopathy iNs.

These results, together with the results of Example 3, demonstrated that a culture supernatant of mouse tauopathy iNs or a tau oligomer isolated from the culture supernatant can be used as an inducer of tau-mediated neurodegeneration.

### [Example 5] Electrophysiological property of mouse tauopathy iN

Properties unique to mouse tauopathy iNs were further searched for. As a result, it was shown, as described below, that nerve excitability has already been significantly increased at the point in time when mouse tauopathy iNs differentiate into neurons.

Tauopathy mouse-derived iPSCs for neural differentiation or normal control mouse-derived iPSCs for neural differentiation were inoculated onto a dish equipped with electrodes, and induced to differentiate into neurons (Figure 5B). As a resulting of measuring a spontaneous firing rate using a MEA system in the iNs of each type of day 8 ([Approach 6]), the spontaneous firing rate of the mouse tauopathy iNs was much higher than the spontaneous firing rate of the mouse normal iNs (Figures 5A and 5C), and the frequency of spikes per cell was approximately 2 times (Figure 5D).

Next, a firing rate after electric stimulation was measured for the iNs of each type of day 8 by use of the whole-cell patch clamp method. As a result, the mouse tauopathy iNs were found to have a significantly higher firing rate than that of the normal iNs (Figures 6A and B). No significant difference in resting membrane potential (Figure 6C) and membrane capacitance (Figure 6D) was found between the iNs.

Change in intracellular calcium concentration caused by electric stimulation was further analyzed by the calcium imaging method. As a result, it was confirmed that depolarization stimulus causes a larger amount of calcium to enter the mouse tauopathy iNs than the normal iNs (Figures 6E and 6F).

These results demonstrated that the mouse tauopathy iNs according to the present disclosure have a high spontaneous firing rate and a high firing rate after stimulation and are thus neurons highly likely to be depolarized.

### [Example 6] Nerve excitability and tau toxicity of mouse tauopathy iN

The relationship between increased nerve excitability in mouse tauopathy iNs and tau oligomerization or cell death caused thereby was analyzed.

AP-5 or CNQX was added to the culture system of mouse tauopathy iNs of day 5. 48 hours later, a culture supernatant and cell extracts were prepared therefrom, and the amount of a tau oligomer, the amount of human tau, and the total amount of tau were analyzed by dot blot (Figures 7A and 7B). Also, AP-5 or CNQX was added to the culture system of mouse tauopathy iNs of day 7. Three days later, a cell survival rate was calculated (Figures 7C and 7D). The administration of any of AP-5 and CNQX drastically decreased the amount of a tau oligomer contained in the culture supernatant (approximately 50% of the negative control, Figure 7B) and drastically elevated the cell survival rate (approximately 2 times the negative control, Figure 7C). By contrast, the administration of CNQX significantly decreased the amount of a tau oligomer contained in the cell extracts (approximately 70% of the negative control, Figure 7B), whereas the administration of AP-5 exhibited no significant change (Figure 7B).

These results indicate that the pharmacological suppression of firing of tauopathy iNs significantly decreases the amount of a tau oligomer present in a medium (= the amount of an extracellularly secreted tau oligomer), but does not always decrease the amount of a tau oligomer present in cells (= the amount of a tau oligomer formed). This demonstrated that the pharmacological suppression of firing of tauopathy iNs suppresses the secretion of a tau oligomer from tauopathy iNs and suppresses neuronal death.

Accordingly, the activity of reducing the excitability of tauopathy iNs was found to serve as an important index for screening for a prophylactic or therapeutic agent for tauopathy. It was also found that the excitability can be evaluated as a spontaneous firing rate, a firing rate after electric stimulation, or change in intracellular calcium concentration after electric stimulation.

### [Example 7] Preparation of tauopathy patient-derived iPSC and preparation of iN from the cell

Subsequently, human iNs were analyzed. iPSCs were prepared from somatic cells of a FTDP-17 patient having a MAPT gene mutation of different type (i.e., a mutation to elevate a 4-repeat tau/3-repeat tau expression ratio) from that in the tauopathy mouse, and iNs were prepared therefrom.

Specifically, fibroblasts derived from the skin collected with the consent from a familial FTDP-17 patient having an intron 10 +14C > T mutation in the MAPT gene were transfected with OCT4, SOX2, KLF4, L-MYC, LIN28, and small haipin RNA for p53 using an episomal vector according to the method described in Okita K, et al., Nat Methods. 2011, 8: 409-12 (see Kondo T, et al., Cell Stem Cell, 2013, 12: 487-96) to establish a tauopathy patient-derived iPSC line. The tauopathy patient-derived iPSCs exhibited embryonic stem cell-like morphology and maintained the expression of an undifferentiation marker (Figure 8A), as with normal subject-derived iPSCs. Also, the tauopathy patient-derived iPSCs were confirmed to retain the mutant MAPT gene (Figure 8B).

iPSC lines for neural differentiation having an insert of the tetracycline-inducible human neurogenin 2 gene in their chromosomes were respectively prepared from the tauopathy patient-derived iPSCs and normal subject-derived iPSCs according to the [Approach 1]. When the expression of the foreign Neurogenin 2 was induced in these two types of iPSCs for neural differentiation according to the [Approach 2], βIII-tubulin-positive cells with extended neurites appeared at day 4; neurite extension and cell body thickening proceeded in the great majority of the cells over days 7 to 10; and approximately 80% or more of the cells exhibited the morphology of mature neurons at day 10 (Figure 8C, upper panels). All of these cells (i.e., human tauopathy iNs and human normal iNs) expressed vGLT1, Satb2, and CaMKII (Figure 8C, lower panels) and were thus confirmed to be glutamatergic cerebral cortex pyramidal cells.

Although the human tauopathy iNs tended to have shorter processes than those of the human normal iNs, no significant difference was found in the number of morphologically mature neurons at day 10 (Figure 8C, upper panels). As a result of performing whole-cell patch clamp at day 10, action potential, albeit weak, was measured in both of the human tauopathy iNs and the human normal iNs. A mature firing pattern was obtained in all assayed cells of the human normal iNs of day 20. This suggests that human iNs start neural activity around day 10, and their functional differentiation finishes up to day 20.

The human normal iNs exhibited no significant difference in the number of living cells between day 8 and day 20 (Figure 8D). By contrast, for the human tauopathy iNs, the number of living cells at day 20 was approximately 70 to 80% of the number of living cells at day 8 (Figure 8D), and approximately 20 to 30% of the cells were found to die up to day 20 (typically, at day 17 or later).

These results demonstrated that when tauopathy patient-derived iPSCs having a mutant MAPT gene are transfected with a foreign neurogenin 2 gene and induced to express the gene, the iPSCs are morphologically matured as cerebral cortex pyramidal cells approximately 10 days later and start neural activity, but then die, as with human normal control-derived iPSCs.

### [Example 8] Pathological condition of tauopathy manifested by human tauopathy iN

Properties exhibited by the human tauopathy iNs were analyzed.

A 4-repeat tau/3-repeat tau mRNA ratio was elevated by 2 times or more in human tauopathy iNs of day 10 as compared with human normal iNs (Figure 9A). Since the total amount of the tau protein expressed was almost the same between the human tauopathy iNs and the human normal iNs, this result indicates that the alternative splicing of exon 10 is inhibited in human tauopathy iNs, as with human tauopathy patients having the mutation.

Also, tau hyperphosphorylation was increased in human tauopathy iNs of day 14 (Figure 9B, middle panel). In immunostaining using a 4-repeat tau-specific antibody, many strongly stained structures were observed in the cell bodies and neurites of the human tauopathy iNs (Figure 9C, right panel), indicating that structures densely packed with 4-repeat tau (probably, aggregates) are produced in human tauopathy iNs. By contrast, any structure strongly stained with the 4-repeat tau-specific antibody was not detected in the human normal iNs (Figure 9C, left panel).

These results demonstrated that in human tauopathy iNs, a 4-repeat tau/3-repeat tau expression level ratio is drastically elevated, tau hyperphosphorylation is promoted, and 4-repeat tau is aggregated, resulting in cell death, as with neurons of patients having the mutant gene.

### [Example 9] Increase of nerve excitability of human tauopathy iN, secretion of tau oligomer, and propagation of neurodegeneration

Human tauopathy iNs were analyzed for the characteristic properties found in the mouse tauopathy iNs.

### Increase of nerve excitability

Change in intracellular calcium concentration caused by electric stimulation was analyzed by the calcium imaging method in human tauopathy iNs and human normal iNs of day 12 ([Approach 7]). As a result, it was shown that depolarization stimulus causes a larger amount of calcium to enter the human tauopathy iNs than the human normal iNs, i.e., nerve excitability is remarkably increased in the human tauopathy iNs (Figures 10A and 10B).

Next, AP-5 or CNQX was added to the culture system of human tauopathy iNs of day 16, and a cell survival rate at day 21 was calculated. The treatment with any of these agents tended to elevate the cell survival rate. Particularly, the treatment with 12.5 µM or 25 µM AP-5 or 25 µM CNQX significantly elevated the cell survival rate (Figures 10C and 10D). This indicated that the suppression of glutamate receptor-mediated spontaneous firing can reduce the cell death of human tauopathy iNs.

These results demonstrated that human tauopathy iNs have very increased excitability and are likely to die due to this increased excitability, as with mouse tauopathy iNs.

### Secretion of tau oligomer

A culture supernatant and cell extracts were prepared from the culture system of human tauopathy iNs or human normal iNs of day 14, and the amount of a tau oligomer and the amount of human tau contained in the culture supernatant or the cell extracts were analyzed by dot blot (Figures 11A and 11B). First, when the cell extracts were compared, there was no significant difference in the amount of human tau between the iNs. However, only a very small amount of the tau oligomer was detected in the normal iNs, whereas a large amount of the tau oligomer was detected in the human tauopathy iNs. Next, when the culture supernatants were compared, there was no significant difference in the amount of human tau between the iNs. However, no tau oligomer was substantially detected in the normal iNs, whereas a significant amount of the tau oligomer was detected in the human tauopathy iNs.

Accordingly, the human normal iNs were found to also slightly produce an intracellular tau oligomer, which is not secreted to the outside of the cells. By contrast, it was shown that the human tauopathy iNs produce a larger amount of an intracellular tau oligomer than that by the normal iNs, and a portion thereof is secreted to the outside of the cells.

Next, the localization of a tau oligomer was analyzed.

Human tauopathy iNs or human normal iNs were double-stained with CTB and an anti-tau oligomer antibody. As a result, only in the human tauopathy iNs, colocalization signals of CTB and the anti-tau oligomer antibody were found on the cell membranes. Accordingly, in the human tauopathy iNs, a tau oligomer was found to bind to lipid raft on the cell membranes of these neurons, as with mouse tauopathy iNs.

Lipid raft was biochemically isolated from human tauopathy iNs or human normal iNs and analyzed for its colocalization with a tau oligomer. Specifically, iNs were homogenized using a 29-gauge needle in a buffer containing 1% Lubrol and a protease inhibitor, and the cell homogenates were adjusted to a sucrose concentration of 40%. Then, a 0.7 ml aliquot thereof was fractionated by the sucrose density-gradient centrifugation method. The sucrose density gradient used was steps of 30% (1.4 ml), 27.5% (0.7 ml), 24% (1.4 ml), and 3% (1 ml) (5-ml tube), and the centrifugation conditions involved 50,000 rpm, 4°C, and 16 hours using Optima-MAX XP1 (Beckman Coulter, Inc.). After the centrifugation, a 0.7 ml aliquot was recovered from the upper layer to obtain 7 fractions (F1 to F7).

Results of analyzing the localization of lipid raft and a tau oligomer in the 7 fractions by Western blot are shown in Figure 11C. The lipid raft (GM1 ganglioside) was recovered into F2 and F3 of the human normal iNs and contained in F2 and F3 as well as in a small amount in F4 of human tauopathy iNs. The tau oligomer in the tauopathy iNs was contained mainly in F3 and also contained in F2 and F4. Thus, the localization of the tau oligomer agreed with that of the lipid raft (arrowheads in the upper panel of Figure 11).

Methyl-P-cyclodextrin (abbreviated to MbCD) is a compound having the action of destroying a lipid raft structure by acting on cholesterol on a cell membrane. When the tauopathy iNs were treated with MbCD and then subjected to the analysis, the tau oligomer was not recovered into F2 to F4 (Figure 11C, lower panel).

These results indicated that a tau oligomer extracellularly secreted from human tauopathy iNs specifically binds to lipid raft on the cell membranes of neurons.

### Propagation of neurodegeneration

A culture supernatant of human tauopathy iNs or human normal iNs of days 14 to 16 was prepared according to the [Approach 4] and added (final concentration: 50% (v/v)) to a medium of mouse normal iNs of day 8, followed by cell survival rate analysis two days later. As a result, only approximately 80% of the number of living cells was observed in the mouse normal iNs supplemented with the culture supernatant of the human tauopathy iNs as compared with the mouse normal iNs supplemented with the culture supernatant of the human normal iNs (Figure 12A).

The culture supernatant of the human tauopathy iNs was further treated with an anti-tau oligomer antibody (immunodepletion) and then added to a medium of human normal iNs, followed by cell survival rate analysis two days later ([Approach 4]). The antibody treatment decreased the amount of a tau oligomer in the culture supernatant to approximately 27% (Figure 12D) and increased the survival rate of the human normal iNs to approximately 130% (Figure 12B). By contrast, in the case of adding a culture supernatant immunodepleted of a tau oligomer using the isotype control antibody instead of the anti-tau oligomer antibody, the number of living cells was not significantly changed as compared with the case of adding the culture supernatant that was not immunodepleted (Figure 12C).

Accordingly, the tau oligomer secreted from the human tauopathy iNs was found to also have the activity of inducing the neurodegeneration of normal neurons. These results demonstrated that a culture supernatant of human tauopathy iNs or a tau oligomer isolated from the supernatant can be used as an inducer of neurodegeneration.

It was further shown that the excitability of neurons is drastically increased by the addition of a culture supernatant of human tauopathy iNs. A culture supernatant of human tauopathy iNs or human normal iNs of days 12 to 14 was added to human normal iNs of day 20, and a spontaneous firing rate was measured using the MEA system of the [Approach 6]. As a result, in the human normal iNs supplemented with the culture supernatant of the human tauopathy iNs, the spontaneous firing rate was remarkably increased (50 times or more that before the addition) approximately 2 minutes after the addition, and this increased state continued (Figures 12E and 12F). By contrast, in the human normal iNs supplemented with the culture supernatant of the human normal iNs, no increase in spontaneous firing rate occurred substantially (Figures 12E and 12F).

Accordingly, the culture supernatant of the human tauopathy iNs was found to have the activity of rapidly converting normal neurons to neurons that are easily excitable as with the human tauopathy iNs themselves. This activity is novel tau toxicity that cannot be predicted from the previously known cytotoxicity of tau.

These results indicated that both of human-derived and mouse-derived neurons having a mutant MAPT gene are more excitable than normal neurons, extracellularly secrete a tau oligomer, and propagate neurodegeneration by increasing the excitability of normal neurons. It was further found that even if the MAPT gene mutation is a mutation to form a mutant tau protein or a mutation to elevate a 4-repeat tau/3-repeat tau expression ratio, neurons having the mutant gene acquire the properties.

### [Example 10] Induction of cell death by artificial tau oligomer

Study was made on whether tau artificially oligomerized *in vitro* could extracellularly exert toxicity to neurons.

P301S mutant human tau produced as a recombinant protein was oligomerized by incubation in the presence of heparin. Specifically, 0.5 mg/ml tau protein was incubated at 37°C for 16 hours in a 10 mM HEPES buffer (pH 7.4) containing 10 µM heparin (Sigma-Aldrich Co. LLC) and 100 mM NaCl (Sahara N. and Takashima A, EMBO J., 26: 5143-52, 2007). Then, the solution was treated at 70°C for 15 minutes and then subjected to SDS-PAGE and silver staining. The obtained image is shown in Figure 13A. As is evident, the tau before the incubation in the presence of the heparin was mostly monomeric and dimeric, whereas the tau after the incubation included a large number of trimeric or larger associates (i.e., tau oligomers). Hereinafter, the tau before the incubation in the presence of the heparin is referred to as a "P301S tau monomer", and the tau after the incubation is referred to as a "P301S tau oligomer".

The P301S tau monomer or the P301S tau oligomer was added to the culture system of human normal iNs, followed by cell survival rate analysis two days later. The survival rate was significantly decreased by the addition of 0.1 µg/ml or 1 µg/ml P301S tau oligomer (Figure 13B), whereas the survival rate was not changed by the addition of 1 µg/ml P301S tau monomer (Figure 13C).

Accordingly, the tau oligomer artificially produced *in vitro* was found to also have the activity of inducing the cell death of normal neurons through its extracellular action. By contrast, monomeric or dimeric tau, if having a mutation to cause tauopathy, was also found to be substantially free from the cell death-inducing activity.

The tau oligomer artificially produced *in vitro* was further found to also have the activity of increasing the nerve excitability of normal neurons.

The P301S tau monomer or the P301S tau oligomer was added (final concentration: 1 µg/ml) to the culture system of human normal iNs, and a spontaneous firing rate was measured using a MEA system ([Approach 6]). As a result, in the human normal iNs supplemented with the P301S tau oligomer, the spontaneous firing rate was drastically increased (approximately 290% of that before the addition) immediately after the addition, and this state continued (Figures 13D and 13E). By contrast, in the human normal iNs supplemented with the P301S tau monomer, the spontaneous firing rate was increased only slightly (approximately 120% of that before the addition) (Figures 13D and 13E).

These results demonstrated that a tau oligomer artificially produced *in vitro* also has the activities of rapidly increasing the excitability of normal neurons through its extracellular action on the cells and inducing cell death, as with a tau oligomer extracellularly secreted by tauopathy iNs. In addition, even mutant tau that causes tauopathy was found to be substantially free from the activity of rapidly increasing excitability and the activity of inducing cell death, in a monomeric or dimeric state.

As shown in Figure 13B, the tau oligomer artificially produced *in vitro* tended to induce cell death, albeit not significant, at 100 ng/ml. By contrast, a culture supernatant of human tauopathy iNs contains a very small amount of a tau oligomer (as a guideline, a supernatant of culture for approximately 5 days contains approximately 1 to 5 ng/ml tau oligomer). Therefore, as is evident, a tau oligomer secreted by tauopathy iNs has much higher activity of increasing nerve excitability and activity of inducing cell death than those of the artificial tau oligomer. This is, for example, because the artificial tau oligomer undergoes no chemical modification such as phosphorylation and does not contain any component other than tau. Accordingly, it is considered that the advancement of biochemical analysis of tau oligomers secreted by human tauopathy iNs may permit production of more highly active tau oligomer derived from recombinant tau.

### [Example 11] Relationship between MAPT gene mutation and ability to secrete tau oligomer

In order to confirm that the properties possessed by neurons having a mutant MAPT gene were attributed to the mutation in the MAPT gene, human iNs in which the gene mutation was corrected were prepared. Specifically, a line of iPSCs in which the MAPT gene mutation in the tauopathy patient-derived iPSCs was corrected to wild type by the substitution of thymine at position 14 of intron 10 in the MAPT gene by cytosine (hereinafter, referred to as "mutation-corrected iPSCs") was prepared (Figure 14A). The base substitution was performed using CRISPR-Cas9 system with the RNA represented by SEQ ID NO: 5 as guide RNA (for the CRISPR-Cas9 system, see Gaj T., et al., Trends Biotechnol., 31: 397-405, 2013; and Doudna J.A., et al., Science, 346, no. 6213, 2014). Results of analyzing the nucleotide sequence of the MAPT gene in the obtained mutation-corrected iPSCs are shown in Figure 14B. As is evident, the position 14 of intron 10 in the MAPT gen was cytosine (i.e., brought back to wild type) in the mutation-corrected iPSCs.

The mutation-corrected iPSCs had almost the same total amount of tau as in human normal control-derived iPSCs and tauopathy patient-derived iPSCs, but had a significantly smaller mRNA level of 3-repeat tau than that of the tauopathy patient-derived iPSCs (Figure 14C) and a 4-repeat tau/3-repeat tau mRNA ratio of approximately 1.0 (i.e., normal level) (Figure 14D).

An iPSC line for neural differentiation was prepared from the mutation-corrected iPSCs according to the [Approach 1]. iNs were prepared from the iPSCs for neural differentiation according to the [Approach 2] (hereinafter, these iNs are referred to mutation-corrected iNs). The mutation-corrected iNs differentiated into glutamatergic cerebral cortex pyramidal cells in almost the same time course as in human normal iNs. Figure 15A shows results of immunostaining human normal iNs, human tauopathy iNs, and mutation-corrected iNs of days 8 and 21 for neuron markers. The number of living cells at day 8 was almost the same among the 3 types of iNs. However, decrease in the number of living cells and neurite retraction were seen in the tauopathy iNs of day 21, and approximately 40% of the iNs died spontaneously (Figure 15B). By contrast, decrease in the number of living cells and neurite retraction were hardly observed in the mutation-corrected iNs even at day 21 (Figures 15A and 15B), demonstrating that the mutation-corrected iNs do not substantially suffer cell death.

A culture supernatant and cell extracts were further prepared from the iNs of each type of day 14, and the content of a tau oligomer was analyzed (Figures 15C and 15D). As a result, in the mutation-corrected iNs, the amount of a tau oligomer in the cell extracts was drastically small as compared with the human tauopathy iNs, and the amount of a tau oligomer in the culture supernatant was close to the value of the human normal iNs (Figure 15D).

These results indicated that the correction of the MAPT gene mutation in tauopathy iNs decreases the amount of a tau oligomer produced so that the amount of an extracellularly secreted tau oligomer becomes almost the same level of normal neurons, and the resulting iNs no longer suffer spontaneous cell death. Accordingly, the cell death depending on the extracellular secretion of tau oligomers and excitability in neurons having a mutant MAPT gene was confirmed to be attributed to the mutation in the MAPT gene.

The results of Examples 1 to 11 demonstrated that neurons having a mutant MAPT gene exhibit increased excitability, extracellularly secrete a tau oligomer in a manner dependent on neural activity, and propagate increased excitability and neurodegeneration to other cells (Figure 16). Accordingly, a compound inhibiting any of this series of processes is expected to suppress the propagation of neurodegeneration in tauopathy and serve as a prophylactic or therapeutic agent for the disease. Furthermore, it was suggested that such a compound can be screened for by using (a) the amount of a tau oligomer in a culture supernatant, (b) the amount of an intracellular tau oligomer, (c) the frequency of depolarization, (d) the activity of decreasing an intracellular calcium ion concentration, or (e) the activity of increasing the number of living cells, as an index in the culture system of neurons having a mutant MAPT gene or the culture system of normal neurons supplemented with a culture supernatant of the cells.

### [Example 12] Screening for compound capable of blocking propagation of tau-mediated neurodegeneration

A compound capable of suppressing the cell death of normal iNs was screened for using a system of adding a culture supernatant of the tauopathy iNs according to the present disclosure to the normal iNs. Specifically, a culture supernatant of mouse tauopathy iNs of days 5 to 7 was added (final concentration: 50% (v/v)) to a medium of mouse normal iNs of day 8. At the same time therewith, each test substance was added thereto (final concentration: 0.1 µM or 1 µM). Then, the cells were cultured for 2 days, and the number of living cells at day 10 was measured. As a result, a larger number of living cells were measured in wells supplemented with 3 types of test substances (compounds A to C) than in control wells supplemented with a vehicle alone (Figure 17). Among them, the wells supplemented with 0.1 µM compound A or compound C had a significant difference from the control wells.

All of these compounds A to C are known as kinase inhibitors, suggesting the possibility that these compounds can block the propagation of tau-mediated neurodegeneration by inhibiting their target kinases.

### INDUSTRIAL APPLICABILITY

A principal challenge to previous drug discovery related to tauopathy has been the suppression of intracellular toxicity exerted by tau. This is because, although tau (monomer) is known to be extracellularly secreted, no toxicity has been found in the extracellular tau monomer.

By contrast, the present inventors have revealed that neurons having a MAPT gene mutation extracellularly secrete a tau oligomer, and the tau oligomer is an actual substance responsible for the propagation of tau-mediated neurodegeneration. Accordingly, the tau oligomer or a culture supernatant containing the oligomer can be used as an inducer of tau-mediated neurodegeneration. Furthermore, a system of adding the inducer to the culture system of normal neurons is a cell culture system that reproduces, for the first time, the propagation of tau-mediated neurodegeneration (in a detectable form), and is a system that can sensitively screen for a compound capable of blocking the propagation. In addition, the screening system is the culture system of normal neurons and can therefore be executed easily even in a facility lacking the technique of preparing pluripotent stem cells.

Thus, by widely spreading the cell culture system and the neurodegeneration inducer disclosed in the present specification, and the screening method using them, it is expected to obtain, in the near future, a prophylactic or therapeutic agent for tauopathy having the mechanism of action based on the suppression of the propagation of tau-mediated neurodegeneration.

## Claims

1. A method for screening for a prophylactic or therapeutic agent for tauopathy, comprising the following steps (1) to (4):
(1) contacting first neurons having a mutant MAPT gene with a test substance, followed by culture;
(2) contacting the culture supernatant of the first neurons obtained in the step (1) with second neurons;
(3) measuring any one index selected from the group consisting of the following (a) to (d) in the second neurons obtained in the step (2):
(a) the amount of an intracellular tau oligomer,
(b) the frequency of depolarization,
(c) an intracellular calcium ion concentration, and
(d) the number of living cells; and
(4) selecting the test substance as a prophylactic or therapeutic agent for tauopathy when the value of (a), (b), or (c) measured in the step (3) after the contact of the first neurons with the test substance in the step (1) is lower than the value obtained without the contact of the first neurons with the test substance in the step (1), and/or when the value of (d) measured in the step (3) after the contact of the first neurons with the test substance in the step (1) is higher than the value obtained without the contact of the first neurons with the test substance in the step (1).

2. A method for screening for a prophylactic or therapeutic agent for tauopathy, comprising the following steps (1) to (4):
(1) contacting a culture supernatant of first neurons having a mutant MAPT gene with second neurons;
(2) further contacting the second neurons with a test substance;
(3) measuring any one index selected from the group consisting of the following (a) to (d) in the second neurons obtained in the step (2):
(a) the amount of an intracellular tau oligomer,
(b) the frequency of depolarization,
(c) an intracellular calcium ion concentration, and
(d) the number of living cells; and
(4) selecting the test substance as a prophylactic or therapeutic agent for tauopathy when the value of (a), (b), or (c) measured in the step (3) after the contact of the second neurons with the test substance in the step (2) is lower than the value obtained without the contact of the second neurons with the test substance in the step (2), and/or when the value of (d) measured in the step (3) after the contact of the second neurons with the test substance in the step (2) is higher than the value obtained without the contact of the second neurons with the test substance in the step (2).

3. The method according to claims 1 or 2, wherein the neurons or the first neurons having a mutant MAPT gene have been differentiated by the expression of foreign Neurogenin 2 from pluripotent stem cells having the mutant MAPT gene.

4. The method according to any one of claims 1 to 3, wherein the second neurons have been differentiated by the expression of foreign Neurogenin 2 from pluripotent stem cells lacking a mutant MAPT gene.

5. The method according to any one of claims 1 to 4, wherein the amount of an intracellular tau oligomer is the amount of the tau oligomer bound to lipid raft on the cells.

6. The method according to any one of claims 1 to 5, wherein the neurons having a mutant MAPT gene express the mutant MAPT gene under the control of a nervous system-specific promoter.

7. The method according to any one of claims 3 to 6, wherein the pluripotent stem cells having the mutant MAPT gene are induced pluripotent stem cells established from somatic cells of a tauopathy patient.

8. The method according to any one of claims 1 to 7, wherein the mutant MAPT gene is a MAPT gene having one or more mutations at exons 9 to 13 or intron 10.

9. The method according to claim 8, wherein the mutations at exons 9 to 13 are mutations to form mutant tau proteins each having one or more amino acid mutations selected from K257T, I260V, G272V, N297K, K280Δ, L284L, N296N, P301L, P301S, S305N, S305S, V337M, E342V, G389R, and R406W.

10. The method according to claim 8, wherein the mutations at intron 10 are one or more mutations in nucleotides at positions 1 to 20 of the intron 10.

11. The method according to claim 2, wherein the step (1) is the step of contacting a tau oligomer isolated from the culture supernatant of the first neurons having a mutant MAPT gene with the second neurons.

## Patentansprüche

1. Verfahren zum Screenen nach einem prophylaktischen oder therapeutischen Agens für Tauopathie, umfassend die folgenden Schritte (1) bis (4):
(1) Inkontaktbringen von ersten Neuronen mit einem mutierten MAPT-Gen mit einer Testsubstanz, gefolgt von Kultivierung;
(2) Inkontaktbringen des Kulturüberstands der im Schritt (1) erhaltenen ersten Neuronen mit zweiten Neuronen;
(3) Messen eines Index, ausgewählt aus der Gruppe, bestehend aus den folgenden (a) bis (d) in den im Schritt (2) erhaltenen zweiten Neuronen:
(a) die Menge an einem intrazellulären tau-Oligomer,
(b) die Depolarisationsfrequenz,
(c) eine intrazelluläre Calciumionen-Konzentration, und
(d) die Anzahl an lebenden Zellen; und
(4) Auswählen der Testsubstanz als ein prophylaktisches oder therapeutische Agens für Tauopathie, wenn der Wert von (a), (b) oder (c), gemessen im Schritt (3) nach dem Kontakt der ersten Neuronen mit der Testsubstanz im Schritt (1), kleiner ist als der ohne den Kontakt der ersten Neuronen mit der Testsubstanz im Schritt (1) erhaltene Wert, und/oder wenn der Wert von (d), gemessen im Schritt (3) nach dem Kontakt der ersten Neuronen mit der Testsubstanz im Schritt (1), größer ist als der ohne den Kontakt der ersten Neuronen mit der Testsubstanz im Schritt (1) erhaltene Wert.

2. Verfahren zum Screenen nach einem prophylaktischen oder therapeutischen Agens für Tauopathie, umfassend die folgenden Schritte (1) bis (4):
(1) Inkontaktbringen eines Kulturüberstands von ersten Neuronen mit einem mutierten MAPT-Gen mit zweiten Neuronen;
(2) weiterhin Inkontaktbringen der zweiten Neuronen mit einer Testsubstanz;
(3) Messen eines Index, ausgewählt aus der Gruppe, bestehend aus den folgenden (a) bis (d) in den im Schritt (2) erhaltenen zweiten Neuronen:
(a) die Menge an einem intrazellulären tau-Oligomer,
(b) die Depolarisationsfrequenz,
(c) eine intrazelluläre Calciumionen-Konzentration, und
(d) die Anzahl an lebenden Zellen; und
(4) Auswählen der Testsubstanz als ein prophylaktisches oder therapeutische Agens für Tauopathie, wenn der Wert von (a), (b) oder (c), gemessen im Schritt (3) nach dem Kontakt der zweiten Neuronen mit der Testsubstanz im Schritt (2), kleiner ist als der ohne den Kontakt der zweiten Neuronen mit der Testsubstanz im Schritt (2) erhaltene Wert, und/oder wenn der Wert von (d), gemessen im Schritt (3) nach dem Kontakt der zweiten Neuronen mit der Testsubstanz im Schritt (2), größer ist als der ohne den Kontakt der zweiten Neuronen mit der Testsubstanz im Schritt (2) erhaltene Wert.

3. Verfahren gemäß Ansprüchen 1 oder 2, wobei die Neuronen oder die ersten Neuronen mit einem mutierten MAPT-Gen aus pluripotenten Stammzellen mit dem mutierten MAPT-Gen durch Expression von fremdem Neurogenin 2 differenziert worden sind.

4. Verfahren gemäß einem der Ansprüche 1 bis 3, wobei die zweiten Neuronen aus pluripotenten Stammzellen ohne ein mutiertes MAPT-Gen durch Expression von fremdem Neurogenin 2 differenziert worden sind.

5. Verfahren gemäß einem der Ansprüche 1 bis 4, wobei die Menge eines intrazellulären tau-Oligomers die Menge des tau-Oligomers, welches an Lipidflöße auf den Zellen gebunden ist, ist.

6. Verfahren gemäß einem der Ansprüche 1 bis 5, wobei die Neuronen mit einem mutierten MAPT-Gen das mutierte MAPT-Gen unter der Kontrolle eines für das Nervensystem spezifischen Promotors exprimieren.

7. Verfahren gemäß einem der Ansprüche 3 bis 6, wobei die pluripotenten Stammzellen mit dem mutierten MAPT-Gen aus somatischen Zellen eines Tauopathie-Patienten gewonnene induzierte pluripotente Stammzellen sind.

8. Verfahren gemäß einem der Ansprüche 1 bis 7, wobei das mutierte MAPT-Gen ein MAPT-Gen mit einer oder mehreren Mutationen bei Exons 9 bis 13 oder Intron 10 ist.

9. Verfahren gemäß Anspruch 8, wobei die Mutationen bei Exons 9 bis 13 Mutationen sind, um mutierte tau-Proteine zu bilden, jedes mit einer oder mehreren Aminosäuremutationen, ausgewählt aus K257T, I260V, G272V, N297K, K280Δ, L284L, N296N, P301L, P301S, S305N, S305S, V337M, E342V, G389R und R406W.

10. Verfahren gemäß Anspruch 8, wobei die Mutationen bei Intron 10 eine oder mehrere Mutationen in Nukleotiden an Positionen 1 bis 20 vom Intron 10 sind.

11. Verfahren gemäß Anspruch 2, wobei der Schritt (1) der Schritt des Inkontaktbringens eines tau-Oligomers, isoliert aus dem Kulturüberstand der ersten Neuronen mit einem mutierten MAPT-Gen, mit den zweiten Neuronen ist.

## Revendications

1. Méthode de criblage pour un agent thérapeutique ou prophylactique pour la tauopathie, comprenant les étapes (1) à (4) suivantes :
(1) la mise en contact de premiers neurones ayant un gène MAPT mutant avec une substance test, suivie par la culture ;
(2) la mise en contact du surnageant de culture des premiers neurones obtenus dans l'étape (1) avec des deuxièmes neurones ;
(3) la mesure d'un indice quelconque choisi parmi le groupe constitué des (a) à (d) suivants dans les deuxièmes neurones obtenus dans l'étape (2) :
(a) la quantité d'un oligomère tau intracellulaire,
(b) la fréquence de dépolarisation,
(c) une concentration d'ions calcium intracellulaire, et
(d) le nombre de cellules vivantes ; et
(4) la sélection de la substance test comme agent thérapeutique ou prophylactique pour la tauopathie lorsque la valeur de (a), (b), ou (c) mesurée dans l'étape (3) après la mise en contact des premiers neurones avec la substance test dans l'étape (1) est inférieure à a valeur obtenue sans la mise en contact des premiers neurones avec la substance test dans l'étape (1), et/ou lorsque la valeur de (d) mesurée dans l'étape (3) après la mise en contact des premiers neurones avec la substance test dans l'étape (1) est supérieure à la valeur obtenue sans la mise en contact des premiers neurones avec la substance test dans l'étape (1).

2. Méthode de criblage pour un agent thérapeutique ou prophylactique pour la tauopathie, comprenant les étapes (1) à (4) suivantes :
(1) la mise en contact d'un surnageant de culture de premiers neurones ayant un gène MAPT mutant avec des deuxièmes neurones ;
(2) la mise en contact supplémentaire des deuxièmes neurones avec une substance test ;
(3) la mesure d'un indice quelconque choisi parmi le groupe constitué des (a) à (d) suivants dans les deuxièmes neurones obtenus dans l'étape (2) :
(a) la quantité d'un oligomère tau intracellulaire,
(b) la fréquence de dépolarisation,
(c) une concentration d'ions calcium intracellulaire, et
(d) le nombre de cellules vivantes ; et
(4) la sélection de la substance test comme agent thérapeutique ou prophylactique pour la tauopathie lorsque la valeur de (a), (b), ou (c) mesurée dans l'étape (3) après la mise en contact des deuxièmes neurones avec la substance test dans l'étape (2) est inférieure à la valeur obtenue sans la mise en contact des deuxièmes neurones avec la substance test dans l'étape (2), et/ou lorsque la valeur de (d) mesurée dans l'étape (3) après la mise en contact des deuxièmes neurones avec la substance test dans l'étape (2) est supérieure à la valeur obtenue sans la mise en contact des deuxièmes neurones avec la substance test dans l'étape (2).

3. La méthode selon la revendication 1 ou 2, dans laquelle les neurones ou les premiers neurones ayant un gène MAPT mutant ont été différenciés par l'expression de Neurogénine 2 étrangère de cellules souches pluripotentes ayant le gène MAPT mutant.

4. La méthode selon l'une quelconque des revendications 1 à 3, dans laquelle les deuxièmes neurones ont été différenciés par l'expression de Neurogénine 2 étrangère de cellules couches pluripotentes manquant un gène MAPT mutant.

5. La méthode selon l'une quelconque des revendications 1 à 4, dans laquelle la quantité d'un oligomère tau intracellulaire est la quantité de l'oligomère tau lié au radeau lipidique sur les cellules.

6. La méthode selon l'une quelconque des revendications 1 à 5, dans laquelle les neurones ayant un gène MAPT mutant exprime le gène MAPT mutant sous le contrôle d'un promoteur spécifique du système nerveux.

7. La méthode selon l'une quelconque des revendications 3 à 6, dans laquelle les cellules souches pluripotentes ayant le gène MAPT mutant sont des cellules souches pluripotentes induites établies à partir de cellules somatiques d'un patient de tauopathie.

8. La méthode selon l'une quelconque des revendications 1 à 7, dans laquelle le gène MAPT mutant est un gène MAPT ayant une ou plusieurs mutations aux exons 9 à 13 ou à l'intron 10.

9. La méthode selon la revendication 8, dans laquelle les mutations aux exons 9 à 13 sont des mutations pour former des protéines tau mutantes ayant chacune une ou plusieurs mutations d'acide aminé choisie(s) parmi K257T, I260V, G272V, N297K, K280Δ, L284L, N296N, P301L, P301S, S305N, S305S, V337M, E342V, G389R, et R406W.

10. La méthode selon la revendication 8, dans laquelle les mutations à l'intron 10 sont une ou plusieurs mutations de nucléotides aux positions 1 à 20 de l'intron 10.

11. La méthode selon la revendication 2, dans laquelle l'étape (1) est l'étape de mise en contact d'un oligomère tau isolé à partir du surnageant de culture des premiers neurones ayant un gène MAPT mutant avec les deuxièmes neurones.
